# EUROPEAN PATENT SPECIFICATION

(11) **EP 3 849 305 B1**
(45) Date of publication and mention of the grant of the patent: **13.12.2023**
(21) Application number: 19860531.3
(22) Date of filing: 11.09.2019
(51) Int. Cl.: A01K 67/027, C07K 14/705, A61K 49/00

(54) **A GENETIC MOUSE MODEL OF AUTOIMMUNE ADVERSE EVENTS AND IMMUNE CHECKPOINT BLOCKADE THERAPY**
GENETISCHES MAUSMODELL FÜR AUTOIMMUNNEBENWIRKUNGEN UND IMMUNCHECKPOINT-BLOCKADETHERAPIE
MODÈLE DE SOURIS GÉNÉTIQUE D'ÉVÉNEMENTS NÉGATIFS AUTO-IMMUNITAIRES ET THÉRAPIE DE BLOCAGE DE POINT DE CONTRÔLE IMMUNITAIRE

(30) Priority: 11.09.2018 US 201862729965 P
(43) Date of publication of application: 21.07.2021
(73) Proprietor: Board of Regents, The University of Texas System, Austin, TX 78701 (US)
(72) Inventor: WEI, Spencer, Houston, TX 77030 (US); ALLISON, James, Houston, TX 77030 (US)
(74) Representative: Vossius & Partner Patentanwälte Rechtsanwälte mbB
(86) International application number: PCT/US2019/050551
(87) International publication number: WO 2020/055962

(56) References cited:
- WO-A1-2016/160721
- WO-A1-2018/041121
- WO-A1-2019/072241
- CN-A- 107 384 959
- KLOCKE KATRIN ET AL: "Induction of autoimmune disease by deletion of CTLA-4 in mice in adulthood", PROCEEDINGS OF THE NATIONAL ACADEMY OF SCIENCES, vol. 113, no. 17, 26 April 2016 (2016-04-26), XP055916241, ISSN: 0027-8424, DOI: 10.1073/pnas.1603892113 Retrieved from the Internet: URL:https://www.ncbi.nlm.nih.gov/pmc/artic les/PMC4855592/pdf/pnas.201603892.pdf>
- WANG JIAN ET AL: "Establishment of NOD- Pdcd1 -/- mice as an efficient animal model of type I diabetes", PROCEEDINGS OF THE NATIONAL ACADEMY OF SCIENCES, vol. 102, no. 33, 16 August 2005 (2005-08-16), pages 11823-11828, XP055916284, ISSN: 0027-8424, DOI: 10.1073/pnas.0505497102
- DU XUEXIANG ET AL: "Uncoupling therapeutic from immunotherapy-related adverse effects for safer and effective anti-CTLA-4 antibodies inCTLA4humanized mice", CELL RESEARCH, SPRINGER SINGAPORE, SINGAPORE, vol. 28, no. 4, 20 February 2018 (2018-02-20), pages 433-447, XP036800645, ISSN: 1001-0602, DOI: 10.1038/S41422-018-0012-Z [retrieved on 2018-02-20]
- DAS RITUPARNA ET AL: "Early B cell changes predict autoimmunity following combination immune checkpoint blockade", THE JOURNAL OF CLINICAL INVESTIGATION, B M J GROUP, GB, vol. 128, no. 2, 1 February 2018 (2018-02-01), pages 715-720, XP009515948, ISSN: 0021-9738, DOI: 10.1172/JCI96798 [retrieved on 2018-01-28]
- YOSHIKO IWAI ET AL: "Cancer immunotherapies targeting the PD-1 signaling pathway", JOURNAL OF BIOMEDICAL SCIENCE, vol. 24, no. 1, 4 April 2017 (2017-04-04), XP055649210, DOI: 10.1186/s12929-017-0329-9
- BRIAN T. FIFE ET AL: "The role of the PD-1 pathway in autoimmunity and peripheral tolerance", ANNALS OF THE NEW YORK ACADEMY OF SCIENCES, vol. 1217, no. 1, 1 January 2011 (2011-01-01), pages 45-59, XP055741061, US ISSN: 0077-8923, DOI: 10.1111/j.1749-6632.2010.05919.x
- WEI SPENCER C. ET AL: "A Genetic Mouse Model Recapitulates Immune Checkpoint Inhibitor-Associated Myocarditis and Supports a Mechanism-Based Therapeutic Intervention", CANCER DISCOVERY, vol. 11, no. 3, 30 November 2020 (2020-11-30), pages 614-625, XP055916192, US ISSN: 2159-8274, DOI: 10.1158/2159-8290.CD-20-0856 Retrieved from the Internet: URL:https://watermark.silverchair.com/614. pdf?token=AQECAHi208BE49Ooan9kkhW_Ercy7Dm3 ZL_9Cf3qfKAc485ysgAAAuMwggLfBgkqhkiG9w0BBw agggLQMIICzAIBADCCAsUGCSqGSIb3DQEHATAeBglg hkgBZQMEAS4wEQQMRRWopwhQAvhnEgqKAgEQgIIClj L6EXc1UZVIAqZZbdggvZo6tgE29c5mM7TBzDv4E9vv TuH90nxK1t0j3AKWpEJ0evKy_9piS55yYhg2T7n2j- TAxqoyyEQP>
- MICHOT J M ET AL: "Immune-related adverse events with immune checkpoint blockade: a comprehensive review", EUROPEAN JOURNAL OF CANCER, ELSEVIER, AMSTERDAM NL, vol. 54, 5 January 2016 (2016-01-05), pages 139-148, XP029401839, ISSN: 0959-8049, DOI: 10.1016/J.EJCA.2015.11.016
- MAK et al.: "An Impaired Immune Tolerance Animal Model Distinguishes the Potential of Troglitazone/Pioglitazone and Tolcapone/Entacapone to Cause IDILI", Toxilogical Sciences, vol. 161, no. 2, 24 October 2017 (2017-10-24), - February 2018 (2018-02), pages 412-420, XP055691034,

## Description

### BACKGROUND

### 1. Field

The present invention is generally in the field of immunology. More particularly, it concerns an animal model for the autoimmune disease etiologies that arise as a result of immune checkpoint blockade therapies as well as methods of using the animal model to screen for agents that mitigate said disease etiologies.

### 2. Description of Related Art

T cell activation is an exquisitely regulated biological process that enables the generation of rapid and highly sensitive responses to foreign antigens while maintaining the ability to distinguish self from non-self and prevent autoimmunity. A key concept underlying this remarkable process is that multiple distinct signals are required to fully activate, or prime, naive T cells. These cues include cognate antigen recognition by the T-cell receptor (TCR) (Signal 1) and CD28 positive co-stimulation (Signal 2). Because CD28 positive co-stimulation is provided by professional antigen presenting cells, this enforces a cell extrinsic requirement for robust T cell activation. The next key step is negative co-stimulation, which is a feedback regulatory mechanism that attenuates T cell activation through inhibition of Signals 1 and 2. *CTLA4* and PD-1 are principal negative costimulatory molecules that attenuate T cell activation through distinct molecular mechanisms. While *CTLA4* attenuates T cell activation via competitive inhibition of CD28 positive co-stimulation, PD-1 primarily acts to inhibit proximal T-cell receptor (TCR) signaling via the phosphatase SHP2 (Chemnitz *et al.,* 2004; Krummel & Allison, 1996; Parry *et al.,* 2005; Walunas *et al.,* 1996). Recent evidence suggests that PD-1 also leads to inhibition of CD28 positive costimulation (Hui *et al.,* 2017) and relatedly, that CD28 signaling is required for effective responses to PD-1 blockade (Kamphorst *et al.,* 2017). This suggests that attenuation of CD28 may be a shared mechanism of PD-1 and *CTLA4* mediated T cell regulation.

T cell activation is generally thought to be governed by a threshold model, in which TCR and costimulatory signals must meet a minimum level to trigger activation. It is unknown whether PD-1 and *CTLA4* negative co-stimulation lead to convergent functional regulation or alternatively, whether they exert distinct regulatory pressures to define the activation threshold. Relatedly, the relative functional contribution of the specific mechanisms of *CTLA4* and PD-1 to T cell attenuation remains unclear. It is possible that these distinct mechanisms converge at the molecular, cellular, and/or tissue level. For example, at the molecular level, *CTLA4* and PD-1 may co-regulate T cell signaling in a cell intrinsic manner through inhibition of CD28. At the cellular level, *CTLA4* and PD-1 attenuate T cells with distinct kinetics with respect to activation and it is unclear whether and how such temporally separated regulation is integrated. Thus, a critical open fundamental question is whether the distinct regulatory mechanisms of *CTLA4* and PD-1 negative co-stimulation functionally interact.

*Anti-CTLA4* and anti-PD-1 therapies are effective in multiple tumor types advanced melanoma and renal cell carcinoma. However, immune checkpoint blockade therapy can induce serious immune-related adverse events as well as *bona fide* autoimmunity, such as myocarditis and type I diabetes in rare instances. There are currently no animal models that faithfully recapitulate the adverse events associated with checkpoint blockade therapy. Treatment of mice with checkpoint blockade antibodies (i.e. anti*-CTLA4,* anti-PD-1) does not lead to significant pathologies and does not faithfully recapitulate the range, severity, and type of immune related adverse events seen in human patients. In particular, these models do not recapitulate the rare autoimmune diseases that are associated with checkpoint blockade. As such, animal models that recapitulate the adverse events associated with checkpoint blockade therapy are needed.

### SUMMARY

The invention relates to the embodiments as characterized in the claims and as described herein below. Provided herein is an animal model that recapitulates autoimmunity induced by checkpoint blockade in human patients. Accordingly, in one embodiment of the present invention, a mouse is provided whose genome comprises: (i) a heterozygous loss-of-function of a *Ctla4* gene and (ii) a homozygous loss-of-function of a *Pdcd1* gene. In one aspect, the mouse has a C57BL/6J genetic background. In some aspects, the mouse is a female mouse. In some aspects, the mouse is a male mouse.

In some aspects, the heterozygous loss-of-function allele of a *Ctla4* gene is further defined as a heterozygous insertion of a neomycin resistance cassette into exon 3 of the *Ctla4* gene. In some aspects, the homozygous loss-of-function allele of the *Pdcd1* gene is further defined as a homozygous deletion of exons 2 and 3 of the *Pdcd1* gene. Furthermore, the mouse may be a *Ctl4*^{tm1All}*Pdcd1*^{tm1.1Shr} mouse.

In some aspects, the mouse suffers from autoimmunity. In certain aspects, the autoimmunity is cardiac autoimmunity or pancreatic autoimmunity. In one aspect, the cardiac autoimmunity is myocarditis. In certain aspects, the myocarditis is fulminant myocarditis. In one aspect, the pancreatic autoimmunity is insulin-dependent diabetes mellitus or lymphocytic pancreatitis. In some aspects, the pancreatic autoimmunity results in pancreatic exocrine destruction or pancreatic islet destruction. In some aspects, the autoimmunity is lymphocytic myocarditis, endarteritis, pancreatic exocrine destruction, pulmonary vasculitis, adipose tissue atrophy (both white and brown), hepatic inflammation, atrophy of female reproductive organs, gastrointestinal tract inflammation, synovitis, or lymphocytic infiltration of the kidney, salivary gland, lacrimal gland, or stomach.

In one embodiment of the present invention, a cell isolated from a mouse of any of the present embodiments is provided. In some aspects, the cell is an immune cell. In some aspects, the cell is a T cell.

In one embodiment of the present invention, methods are provided for screening at least one candidate agent in a mouse of the present embodiments, the methods comprising administering one or more candidate agent to the mouse. In some aspects, the methods further comprise screening the at least one candidate agent in a mouse comprising (i) a homozygous wild-type *Ctla4* gene and (ii) a homozygous loss-of-function of a *Pdcd1* gene; a mouse comprising (i) a homozygous wild-type *Ctla4* gene and (ii) a homozygous wild-type *Pdcd1* gene; a mouse comprising (i) a homozygous wild-type *Ctla4* gene and (ii) a heterozygous loss-of-function allele of a *Pdcd1* gene; a mouse comprising (i) a heterozygous loss-of-function allele of a *Ctla4* gene and (ii) a homozygous wild-type *Pdcd1* gene; and/or a mouse comprising (i) a heterozygous loss-of-function allele of a *Ctla4* gene and (ii) a heterozygous loss-of-function of a *Pdcd1* gene; and/or a mouse comprising (i) a heterozygous loss-of-function allele of a *Ctla4* gene and (ii) a homozygous loss-of-function allele of a *Pdcd1* gene.

In some aspects, the at least one candidate therapeutic agent is screened for its ability to mitigate an immune-related adverse event or immune-related condition. In some aspects, mitigating an immune-related adverse event or immune-related condition is further defined as preventing the development of the immune-related adverse event or immune-related condition. In some aspects, mitigating an immune-related adverse event or immune-related condition is further defined as decreasing the severity of the immune-related adverse event or immune-related condition. In some aspects, mitigating an immune-related adverse event or immune-related condition is further defined as mitigating the mortality resulting from *Ctla4* haploinsufficiency. In some aspects, mitigating an immune-related adverse event or immune-related condition is further defined as mitigating a systemic immune-related adverse event or immune-related condition. In some aspects, mitigating an autoimmunity is further defined as mitigating organ- or tissue-specific autoimmunity. In some aspects, mitigating an immune-related adverse event or immune-related condition is further defined as decreasing the severity of the immune-related adverse event or immune-related condition. In some aspects, mitigating an immune-related adverse event or immune-related condition is further defined as decreasing the frequency at which the immune-related adverse event or immune-related condition manifests in the population of mice. In some aspects, mitigating an immune-related adverse event or immune-related condition is further defined as slowing the development, or time to onset, of the immune-related adverse event or immune-related condition. In some aspects, screening a candidate therapeutic agent is defined as testing the efficacy of the candidate therapeutic agent.

In some aspects, the immune-related adverse event or immune-related condition is inflammation, such as, for example, acute inflammation or chronic inflammation. In some aspects, the immune-related adverse event or immune-related condition is autoimmunity or an autoimmune condition. In some aspects, the immune-related adverse event or immune-related condition comprises an immune-related adverse event or immune-related condition that mimics an immune-related adverse event or an autoimmunity induced by a checkpoint blockade therapy in humans. In certain aspects, the immune-related adverse event or immune-related condition is cardiac autoimmunity or pancreatic autoimmunity. In one aspect, the cardiac autoimmunity is myocarditis, such as, for example, fulminant myocarditis. In one aspect, the pancreatic autoimmunity is insulin-dependent diabetes mellitus.

In some aspects, the candidate agent is a *CTLA4*-immunoglobulin fusion protein (e.g., abatacept or a murine version thereof), a steroid, an agent that depletes a specific population of immune cells (e.g., anti-CD4 antibody to deplete CD4 T cells or anti-CD20 monoclonal antibody (e.g., rituximab) to deplete B cells), a cytokine modulating agent (e.g., toclizumab or a murine version thereof), or an immunosuppressive agent. In some aspects, the candidate agent is an anti-cancer therapy (e.g., chemotherapy, radiation, surgery, kinase inhibitors, immunotherapies, anti-TIM3, anti-OX40, oncolytic viruses, bispecific antibodies) and the method is screening for additional adverse events that occur in mice suffering from autoimmunity that mimics an immune-related adverse event. The screening may identify therapeutic agents, that when combined with immune checkpoint blockade, have an unfavorable risk profile for the development of autoimmunity or immune-related adverse events. In some aspects, the candidate agent is a pathogen (e.g., commensal or infectious), stress, an injury, and/or a diet. In some aspects, the candidate agent is a tumor cell, such as a syngeneic tumor cell (e.g., B16 melanoma, MC38 colon carcinoma, Lewis lung carcinoma). The tumor cell may be engrafted into the mouse and the effect of the resulting tumor on the immune-related adverse events may be characterized. Tumor properties tested may include total tumor burden, tumor lysis resulting from therapy, release of tumor-associated antigens (e.g., injection of irradiated tumor cells as a tumor immunization), or specific properties of the tumor (e.g., specific mutations or activity of specific genes).

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only.

### BRIEF DESCRIPTION OF THE DRAWINGS

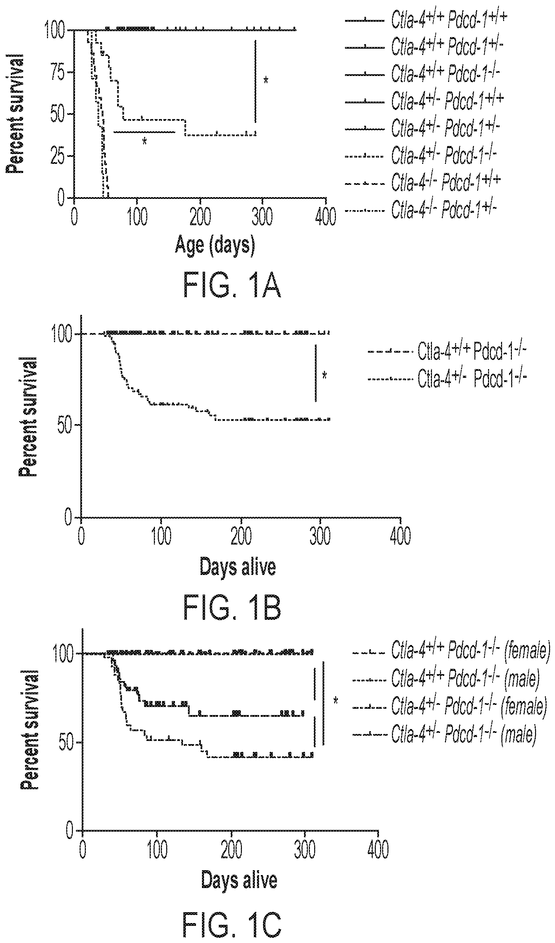

FIGS. 1A-C. Genetic interaction between ***Ctla4* and *Pdcd1* reveals lethal haploinsufficiency phenotypes.** (FIG. 1A) Kaplan-Meier survival curve of transgenic C57BL6/J mice harboring *Ctla4* and *Pdcd1* knockout alleles (n = 138 total mice with n = 5 *Ctla4*^{+/+} *Pdcd1*^{+/+}*,* n = 6 *Ctla4*^{*+*/}*⁺ Pdcd1*^{+/-}*,* n = 32 *Ctla4*^{+/+} *Pdcd1*^{*-*/-}*,* n = 10 *Ctla4*^{+/-} *Pdcd1*^{+/+}, n = 50 *Ctla4*^{+/}*⁻ Pdcd1*^{+/}*⁻,* n = 14 *Ctla4*^{+/-} *Pdcd1*^{*-*/}*⁻,* n = 14 *Ctla4*^{-/-} *Pdcd1*^{+/+}*,* and n = 7 *Ctla4*^{*-*/}*⁻ Pdcd1*^{+/*-*} mice). Mice were derived from an intercross of *Ctla4*^{+/-} *Pdcd1*^{+/*-*} mice in which *Pdcd1* and *Ctla4* loss of function alleles are in *trans.* Individual mice were censored if used for breeding or alive at the time of data analysis. Death events were defined as mice found dead or identified by veterinary staff as requiring euthanasia. (FIG. 1B) Kaplan-Meier survival curve of *Ctla4*^{+/-} *Pdcd1*^{-/*-*} (n = 102) and littermate *Ctla4*^{+/+} *Pdcd1*^{-/*-*} (n = 106) mice derived from a breeding cross of male *Ctla4*^{+/}*⁻ Pdcd1*^{-/*-*} and female *Ctla4*^{*+*/}*⁺ Pdcd1*^{-/*-*} mice. (FIG. 1C) Kaplan-Meier survival curve of *Ctla4*^{+/-} *Pdcd1*^{-/*-*} and littermate *Ctla4*^{*+*/}*⁺ Pdcd1*^{*-*/*-*} mice stratified by mouse sex (n = 24 and 29, male and female *Ctla4*^{+/-} *Pdcd1*^{*-*/}*⁻;* n = 38 and 22, male and female *Ctla4*^{+/+} *Pdcd1^{-l-}).* Pairwise comparisons with Mantel-Cox Log-rank p-values less than 0.05 are denoted.

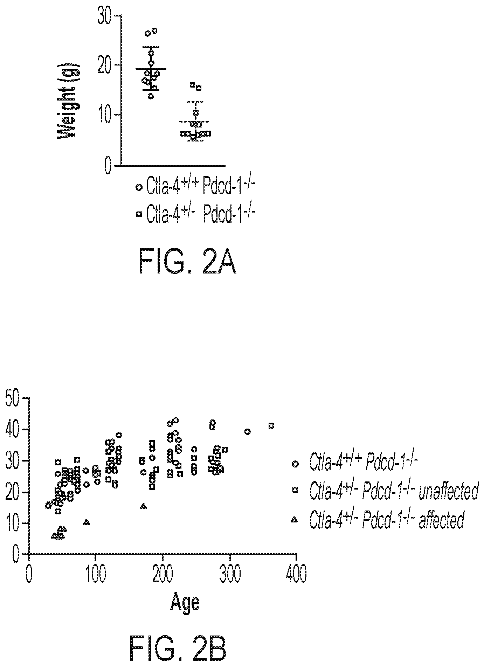

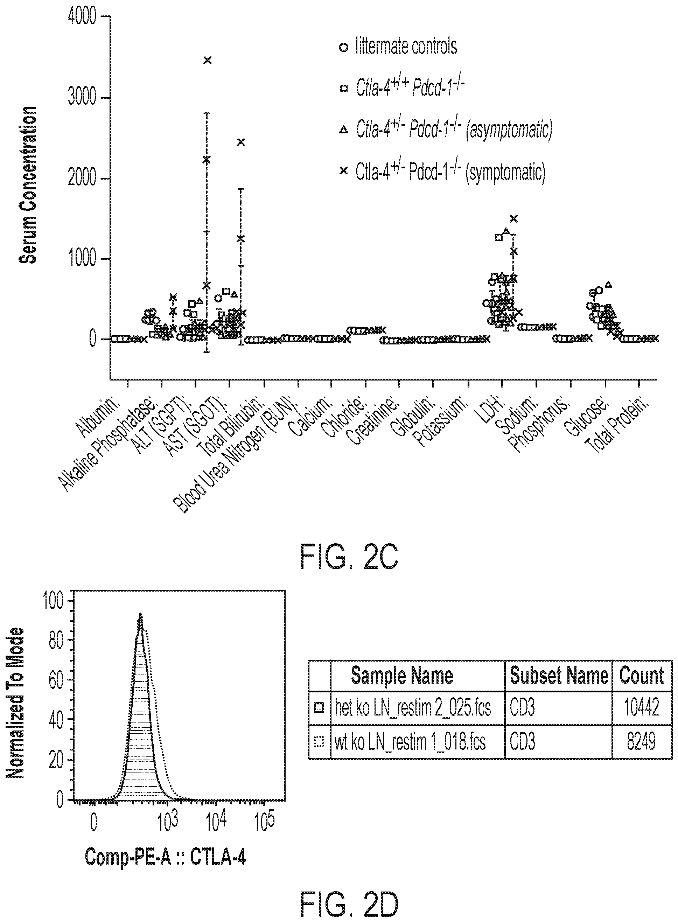

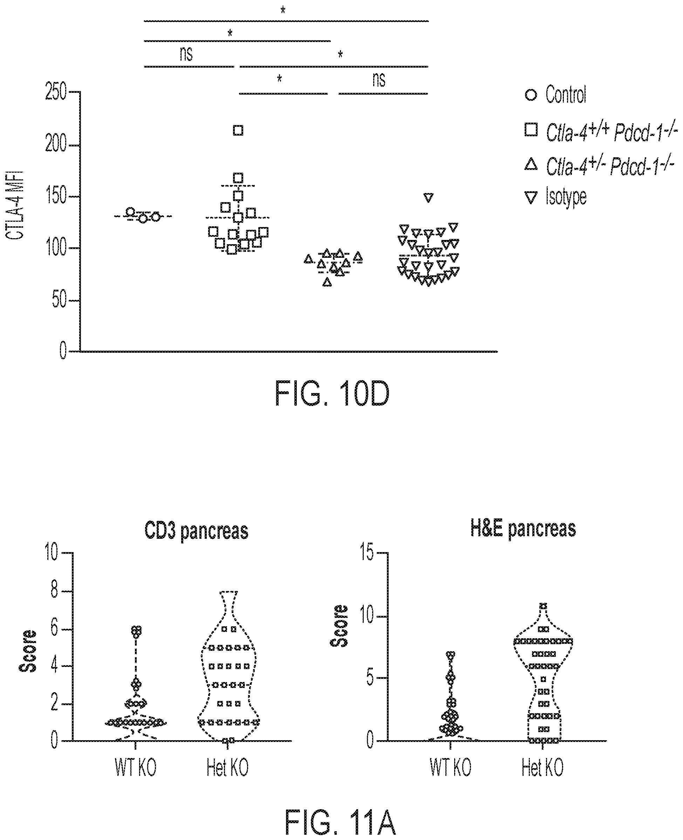

FIGS. 2A-D. Mono-allelic loss of ***Ctla4* in the absence of PD-1 leads to mortality with 50% penetrance.** (FIG. 2A) Total body weight of symptomatic *Ctla4*+/*- Pdcd1-*/*-* and littermate control *Ctla4*+/+ *Pdcd1-*/*-* mice. (FIG. 2B) Total body weight of *Ctla4*+/*- Pdcd1-*/*-* and littermate control *Ctla4*+/+ *Pdcd1-*/*-* mice plotted as a function of age. *Ctla4*+/*- Pdcd1-*/*-* mice identified as requiring euthanasia are identified. Weights of *Ctla4*+/*- Pdcd1-*/*-* mice that were found dead without display of any symptoms were not able to be recorded and thus are not included here. (FIG. 2C) Serum chemistry of phenotypically unaffected aged 4-10 month old *Ctla4*+/*- Pdcd1-*/*-,* littermate control *Ctla4*+/+ *Pdcd1-*/*-* mice, and symptomatic *Ctla4*+/*- Pdcd1-*/*-* mice was performed. Significantly elevated levels of ALT, AST, and LDH were observed in symptomatic *Ctla4*+/*- Pdcd1-*/*-* mice. (FIG. 2D) A representative plot of total *CTLA4* protein levels in *in vitro* stimulated T cells assessed by flow cytometry. *CTLA4* expression levels in T cells derived from *Ctla4*+/+ *Pdcd1-*/*-* mice are plotted as a dotted line and that of *Ctla4*+/*- Pdcd1-*/*-* T cells plotted as a solid line. Quantitative data shown in FIG. 10D.

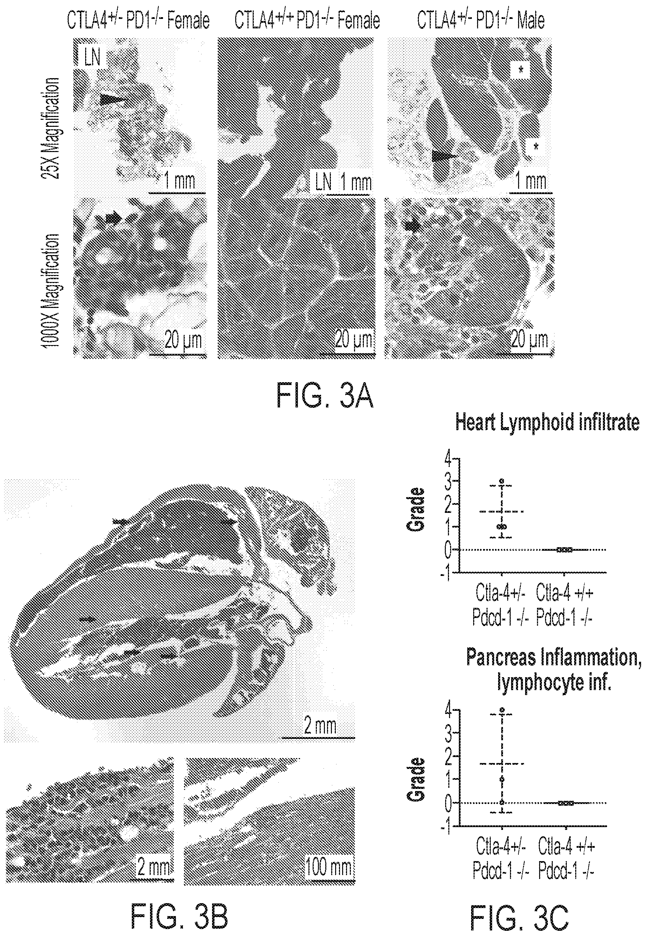

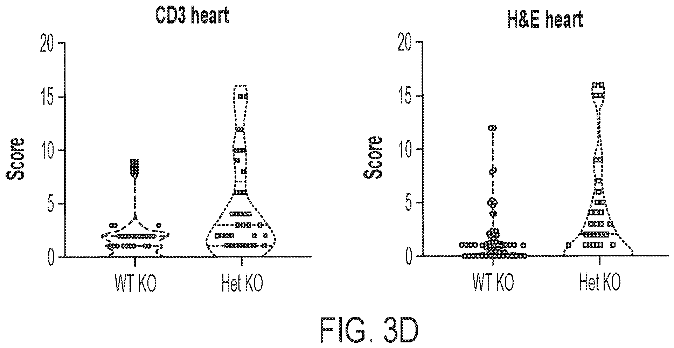

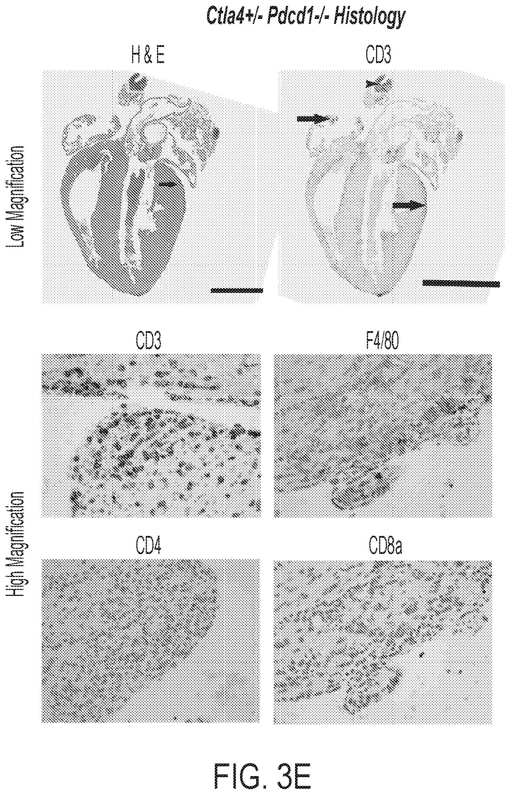

FIGS. 3A-E. ***Ctla4*^{+/-} *Pdcd1*^{-/*-*} mice develop autoimmunity in multiple tissues.** (FIG. 3A) Images of H&E stained FFPE pancreatic tissue sections from *Ctla4*^{+/}*⁻ Pdcd1*^{-/*-*} and *Ctla4*^{*+*/}*⁺ Pdcd1*^{-/*-*} mice. (FIG. 3B) Images of H&E stained FFPE heart tissue sections from *Ctla4*^{+/-} *Pdcd1*^{-/*-*} and *Ctla4*^{*+*/}*⁺ Pdcd1*^{-/*-*} mice. (FIG. 3C, upper panel) Quantification of lymphoid infiltrate score in heart and pancreas tissue. (FIG. 3C, lower panel) Quantification of total T cells in pancreatic and heart tissue sections. (FIG. 3D) Graph shows quantification of total CD3 and lymphoid infiltrate scores in heart tissue. (FIG. 3E) Panels show representative histology images of heart tissue from Ctla4^{+/-} Pdcd1^{-/-} mice. H&E staining (top left) and CD3 immunohistochemistry (top right) images at low magnification are displayed. Arrows denote example areas of immune infiltration. Bottom rows: histology images at high magnification of immunohistochemistry staining of CD3 (T cell marker), CD4 (T cell marker), CD8 (T cell marker), and F4/80 (macrophage marker). This is an example of infiltration of each of these cell subtypes into the heart tissue of Ctla4^{+/-} Pdcd1^{-/-} mice.

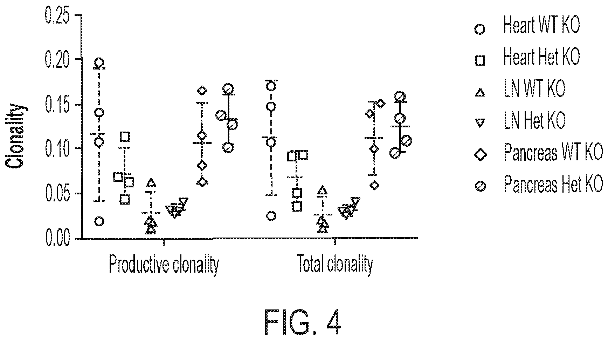

**FIG. 4****. Molecular characterization of the immune response in *Ctla4*^{+/}*⁻ Pdcd1*^{-/*-*} mice.** T cell clonality assessed by TCR sequencing in lymph node, heart, and pancreatic tissue from *Ctla4*^{+/}*⁻ Pdcd1*^{-/}*⁻ Pdcd1*^{+/*-*} and *Ctla4*^{*+*/}*⁺ Pdcd1*^{-/*-*} mice.

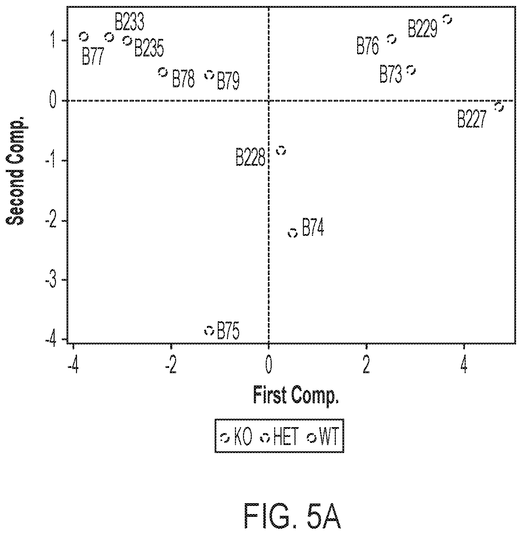

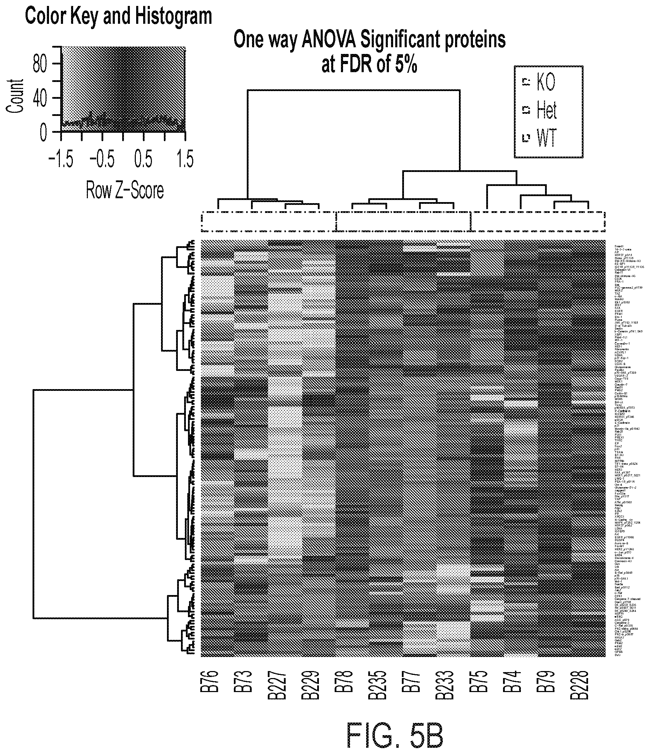

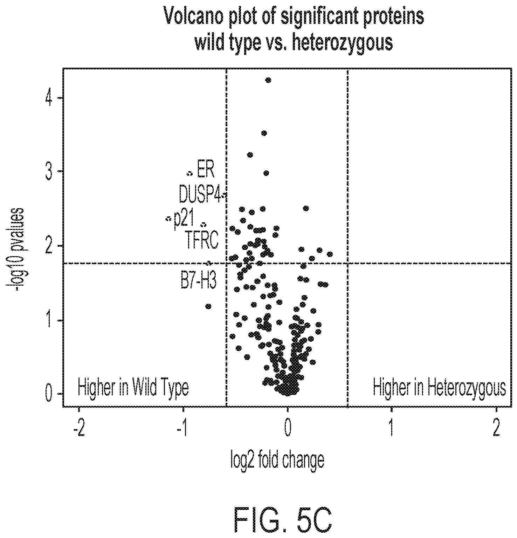

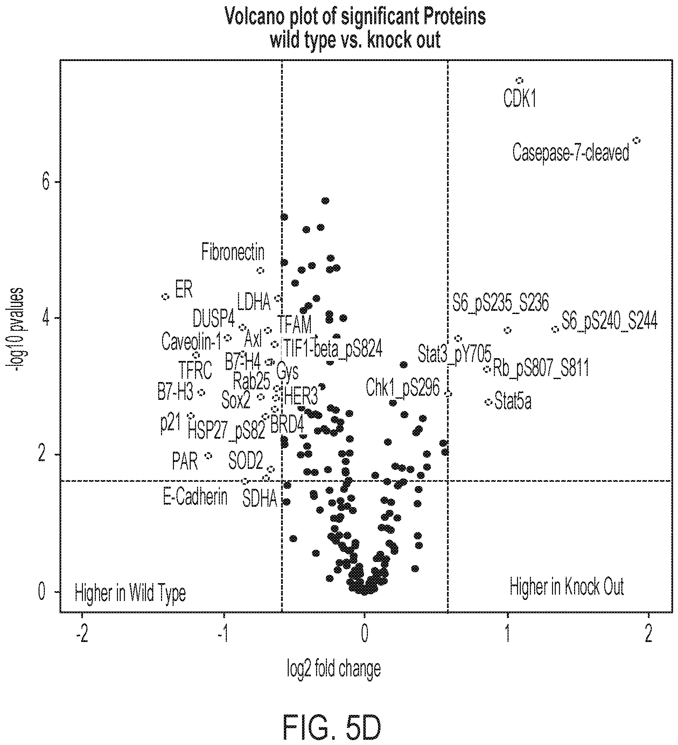

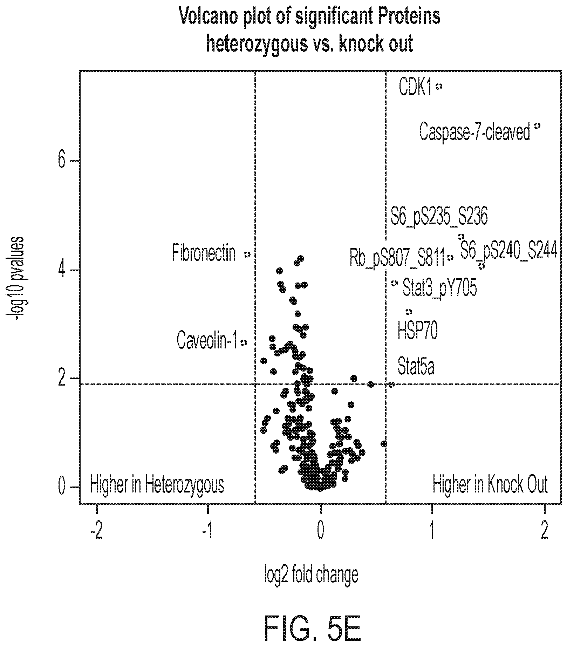

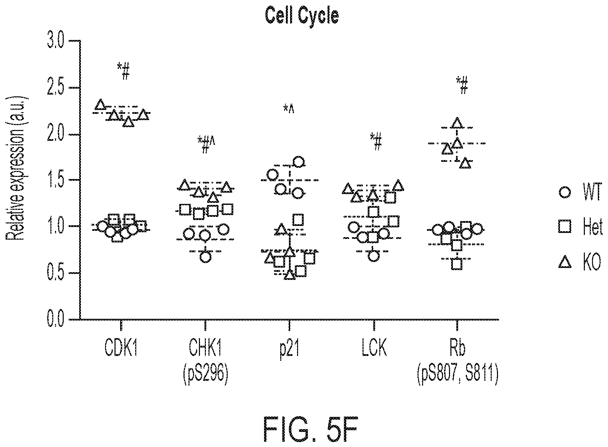

FIGS. 5A-F. Proteomic analyses reveal very subtle molecular changes due **to single copy loss of *Ctla4.*** (FIG. 5A) Principal component analysis plot of RPPA analysis of lymph nodes from *Ctla4*^{*-*/}*⁻, Ctla4*^{+/-}*,* and *Ctla4*^{+/+} mice. (FIG. 5B) The expression of significantly modulated proteins displayed as a heatmap organized by two-way unsupervised hierarchical clustering. (FIG. 5C-E) Volcano plots of protein expression comparing wild-type versus heterozygous mice (FIG. 5C), wild-type versus *Ctla4* knockout (FIG. 5D), and heterozygous versus *Ctla4* knockout mice (FIG. 5E). (FIG. 5F) Expression values of specific proteins associated with cell cycle plotted as on a per mouse basis. Proteins with Tukey's multiple comparison p < 0.05 between wild-type and heterozygous mice are denoted.

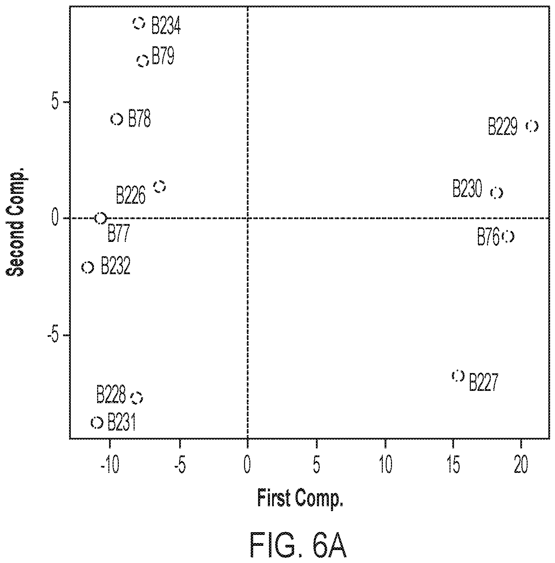

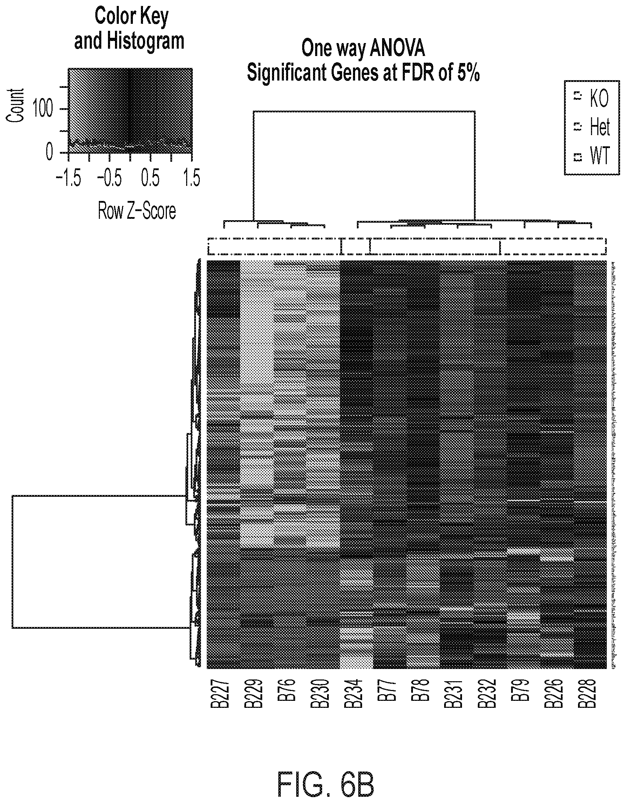

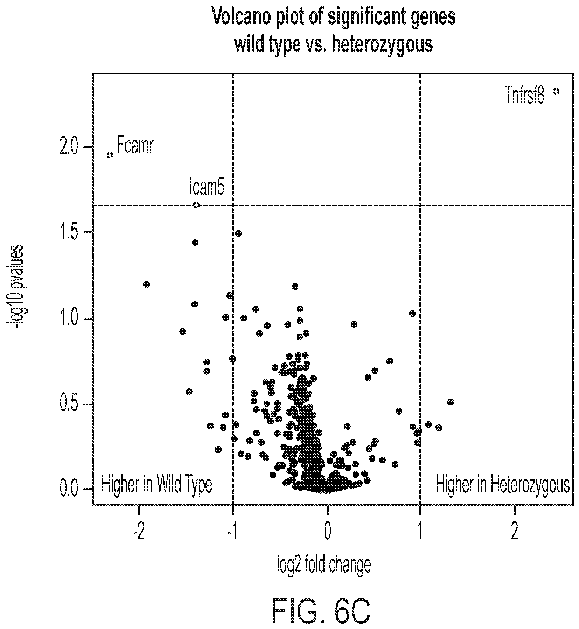

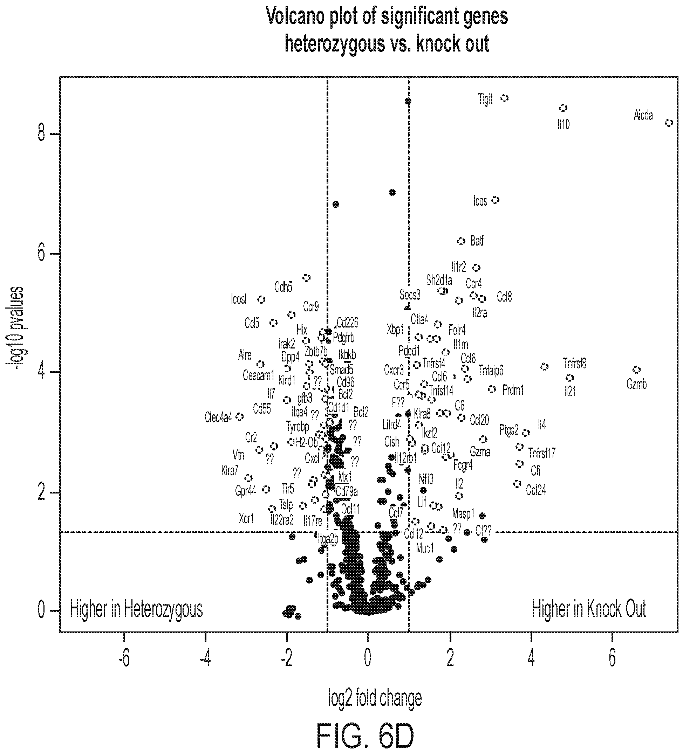

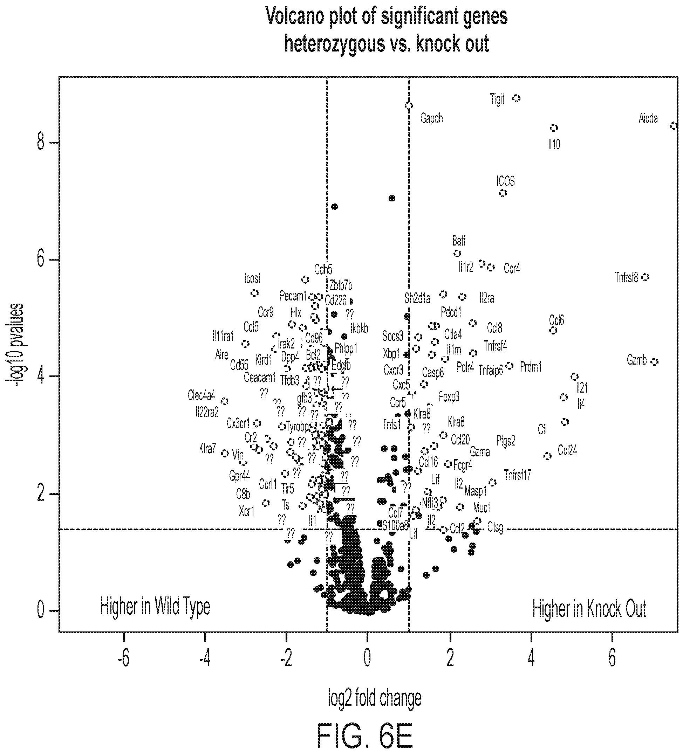

FIGS. 6A-E. Transcriptional analyses reveal little to no changes due to **single copy loss of *Ctla4* in context of functional PD-1.** (FIG. 6A) Principal component analysis plot of Nanostring gene expression analysis of lymph nodes from *Ctla4*^{*-*/*-*} and littermate control mice (all homozygous wild-type *Pdcd1*). (FIG. 6B) The expression of significantly modulated proteins displayed as a heat map organized by two-way unsupervised hierarchical clustering. (FIGS. 6C-E) Volcano plots of protein expression comparing wild-type versus heterozygous mice (FIG. 6C), wild-type versus *Ctla4* knockout (FIG. 6D), and heterozygous versus *Ctla4* knockout mice (FIG. 6E).

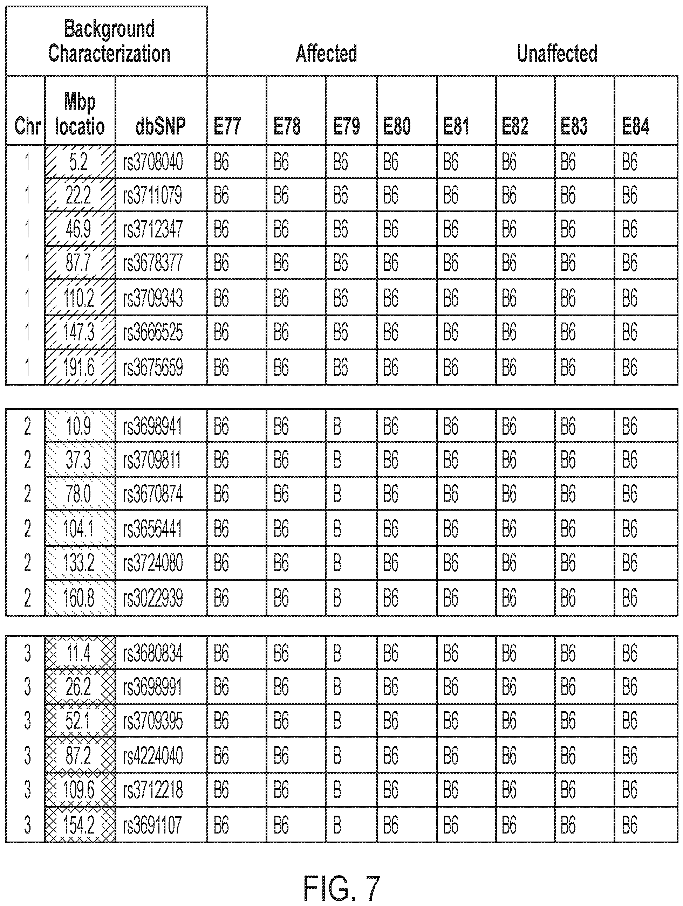

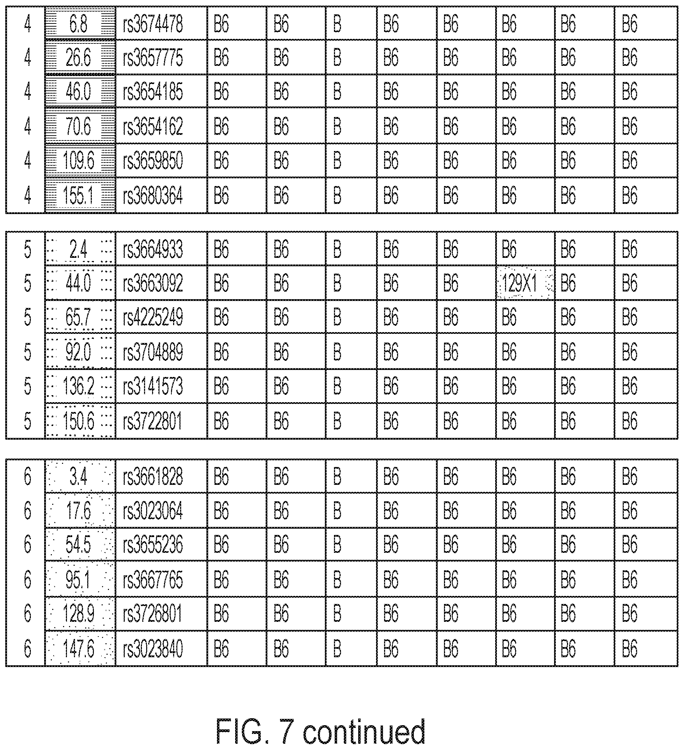

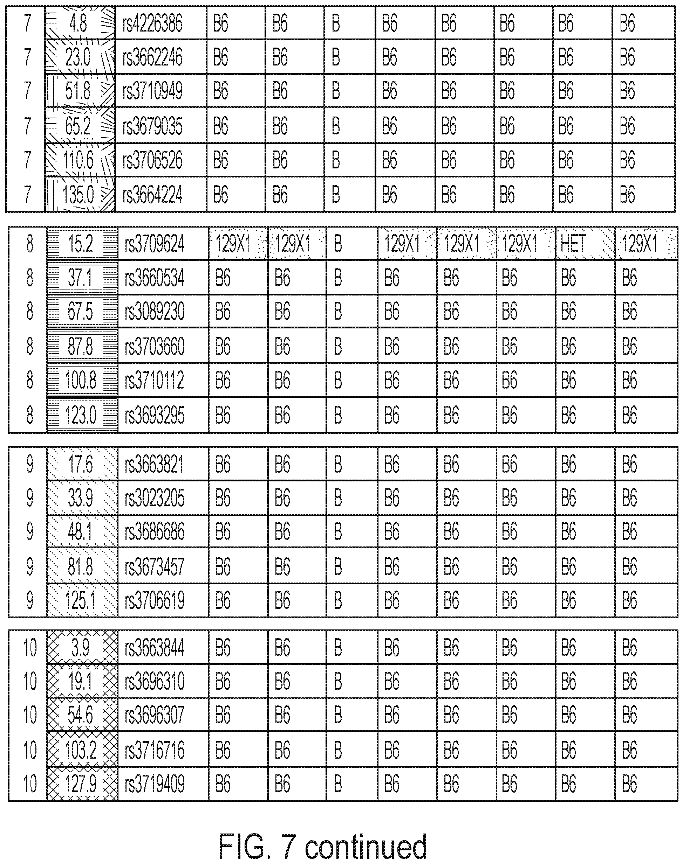

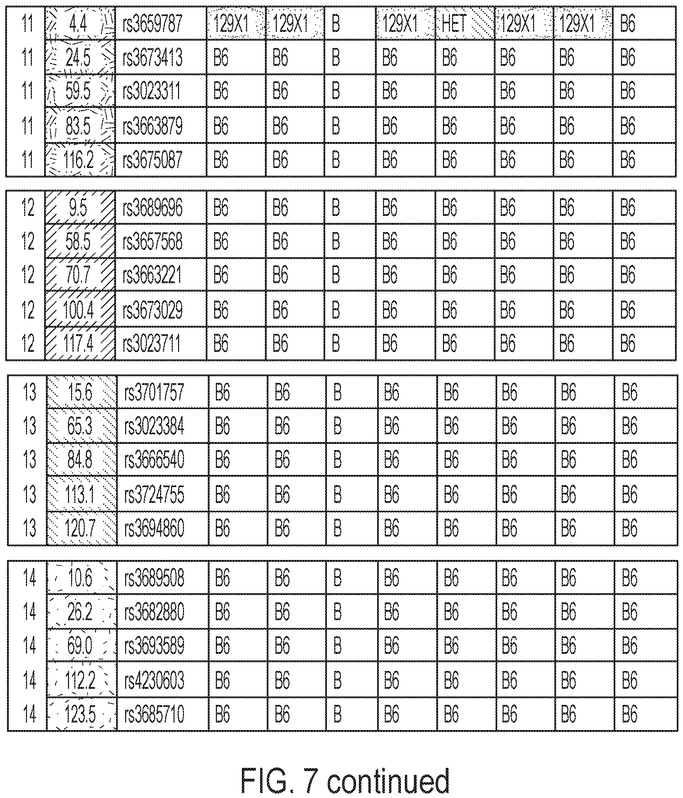

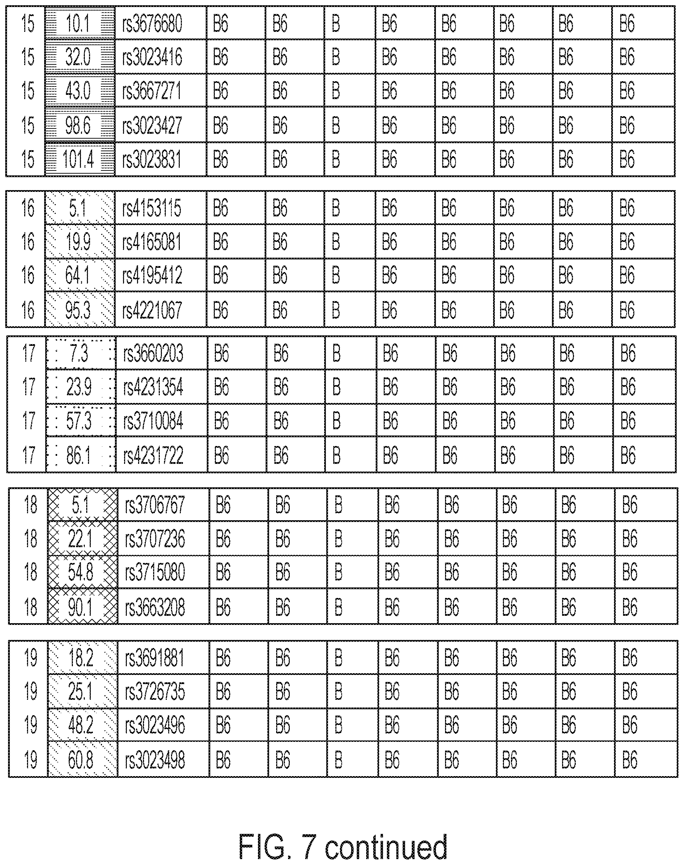

**FIG. 7****. *Ctla4*^{+/-} *Pdcd1*^{*-*/*-*} mice are on a nearly pure C57BL6/J strain background and no segregating SNPs are associated with pathology.** 100-SNP panel assessing strain background of pathogenic (affected) and non-pathogenic (unaffected) *Ctla4*^{+/}*⁻ Pdcd1*^{-/*-*} mice. All tested mice harbored 97-100% C57BL6/J alleles.

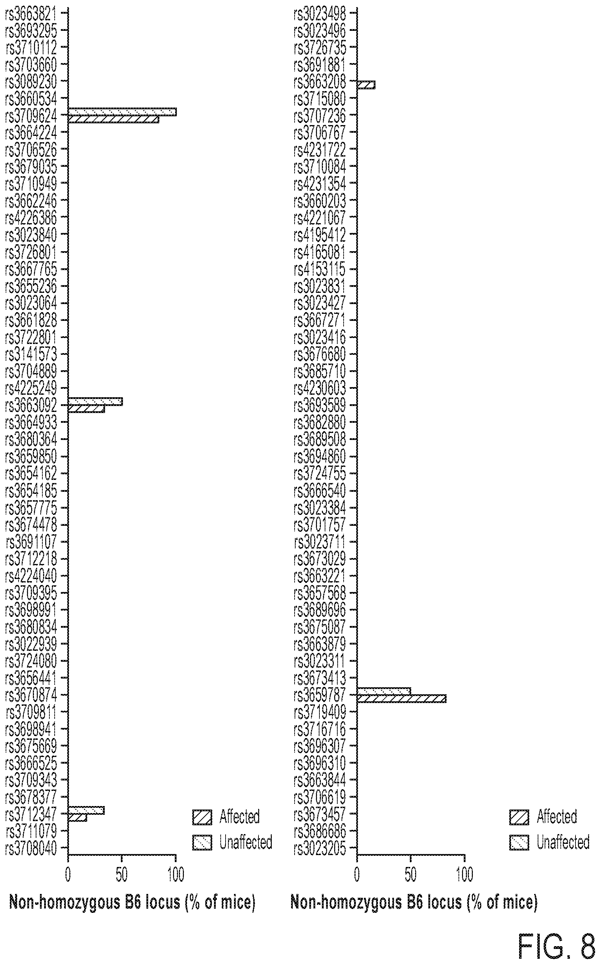

**FIG. 8****. No segregating SNPs are associated with pathology of *Ctla4*+/-*Pdcd1-l-* mice.** The frequency of non-homozygous B6 alleles across all mice are displayed for each SNP tested, non-homozygous B6 locus is defined as either heterozygous for B6/129 or homozygous for 129 alleles.

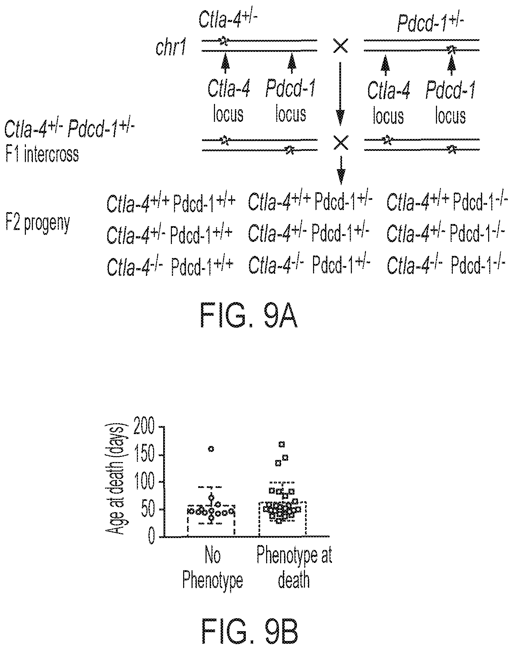

FIG 9A-B. Generation and characterization transgenic mice with **compound loss of function alleles of Ctla4 and *Pdcd1.*** (FIG 9A) Schematic of the breeding scheme to generate all potential combinations of Ctla4 and *Pdcd1* loss of function mutant alleles. (FIG 9B) Age of death of class I (mice that presented clinical symptoms prior to death) and class II (mice that died with prior presentation of symptoms) *Ctla4+*/*- Pdcd1-*/*-* mice.

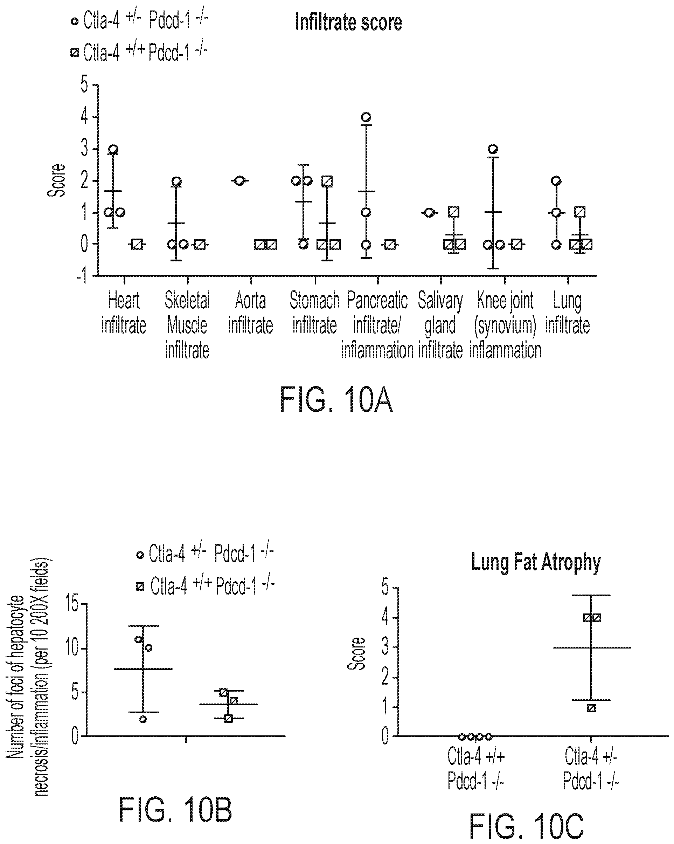

FIG 10A-D. Broad histological characterization of ***Ctla4+*/*- Pdcd1-*/*-* mice reveals multi-tissue autoimmunity.** (FIG. 10A) Scores of immune infiltration in the tissues denoted based on histological analyses of *Ctla4*+/*- Pdcdl-*/*-* and littermate control mice. (FIG. 10B) Number of foci of hepatic necrosis and/or inflammation per area of liver tissue analyzed by histology. (FIG. 10C) Score of lung adipose tissue atrophy in *Ctla4*+/*- Pdcd1-*/*-* and littermate control mice. (FIG. 10D) Quantitation of total CTLA-4 protein levels in *in vitro* stimulated T cells assessed by flow cytometry. CTLA-4 expression levels in T cells derived from *Ctla4*^{+/+} *Pdcd1*^{*-*/*-*} mice are plotted as a dotted line and that of *Ctla4*^{+/}*⁻ Pdcd1*^{*-*/*-*} T cells plotted as a filled solid line.

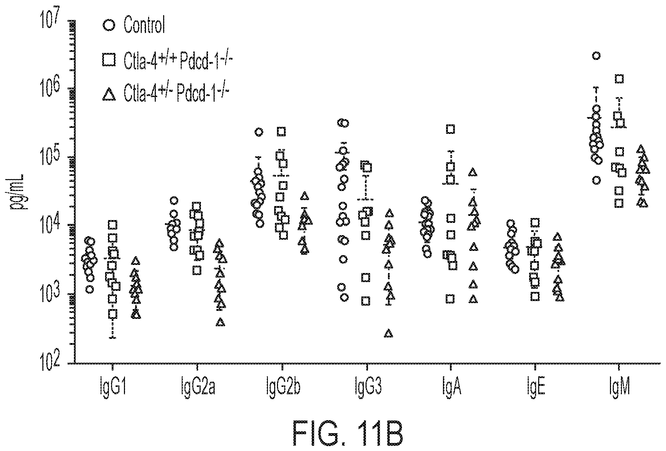

FIG 11A-B. Pathology of cardiac and pancreatic tissue in ***Ctla4*^{+/}*⁻ Pdcd1*^{*-*/*-*} mice.** (FIG. 11A) Graphs show additional histology quantification from pancreas tissues. (FIG. 11B) Graph shows quantitation of serum antibody concentrations.

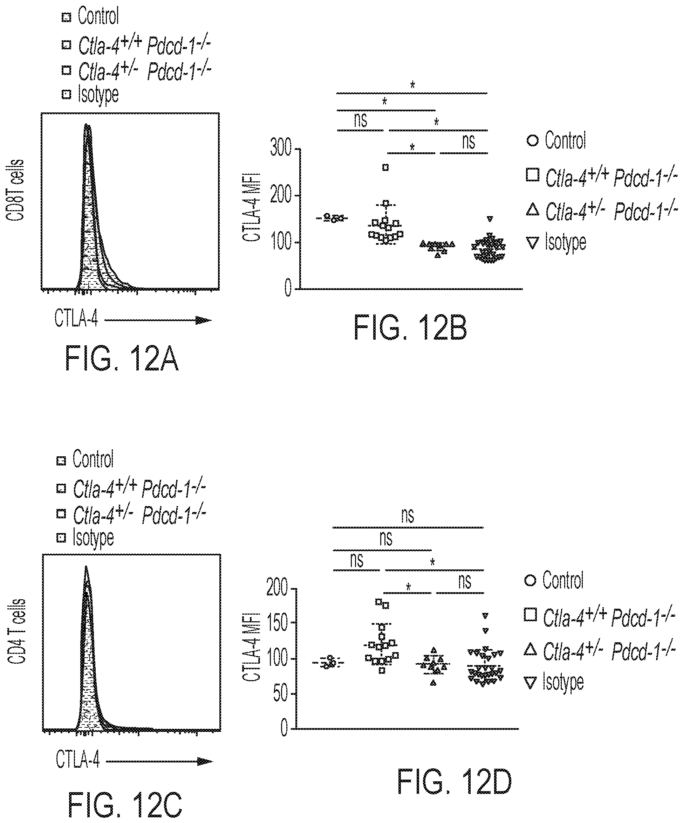

FIG 12A-D. Mono-allelic loss of ***Ctla4* leads to decreased CTLA-4 protein.** (FIG. 12A-D) Flow cytometry analysis total CTLA-4 in CD8 (A and B) and CD4 (C and D) T cells derived from *Ctla4*^{+/}*⁻ Pdcd1*^{*-*/}*⁻,* littermate *Ctla4*^{*+*/}*⁺ Pdcd1*^{*-*/}*⁻,* and C57BL6/J mice.

### DETAILED DESCRIPTION

T cell activation is tightly regulated via a wide range of mechanisms including negative co-stimulation, but the extent to which individual molecular mediators functionally interact remains unclear. *CTLA4* and PD-1 are key negative regulators of T cell activation that utilize distinct molecular and cellular mechanisms. *CTLA4* and PD-1 are both T cell negative costimulatory molecules whose function is to attenuate T cell activity. These molecules utilize distinct molecular mechanisms to carry out these functions. These molecular mechanisms are mediated by largely distinct cell signaling pathways (Wei *et al.,* 2018). The primary function of *CTLA4* is to compete for binding of B7 ligands (B7-1/CD80, B7-2/CD86), which leads to a reduction in CD28 positive costimulation and downstream PI3K/AKT signaling. The primary function of PD-1 is to inhibit proximal T cell receptor signaling upon binding to its ligands PD-L1/PD-L2; however, inhibition of CD28 signaling has also been reported as a significant function of PD-1 (Hui *et al.,* 2017). Antibody-mediated blockade inhibits these functions by preventing ligand binding. This loss of signaling capacity and the consequent downstream biological events can be modeled by genetic means through the combination of loss of function alleles of *Ctla4* and *Pdcd1.*

The inventors sought to understand whether regulatory mechanisms imposed by *CTLA4* and PD-1 are functionally independent or dependent and found evidence of genetic interaction between *Ctla4* and *Pdcd1* (encoding PD-1) in mice. Mono-allelic loss of *Ctla4* in the context of complete genetic absence of *Pdcd1* led to death in approximately half of mice. Mortality was caused by autoimmunity in multiple tissues, including pancreas and heart (see, *e.g.,* FIG. 3E). In contrast, no deaths or severe phenotypes were observed in littermate *Ctla4*^{+/+} *Pdcd1*^{*-*/*-*} or *Ctla4*^{+/}*⁻ Pdcd1*^{+/}*⁻.* These data reveal that *Ctla4* exhibits conditional haploinsufficiency in the context of a *Pdcd1* null background. Together, these findings indicate that *CTLA4* and PD-1 functionally interact and support a threshold model in which negative costimulatory molecules attenuate T cell activation in a dose-dependent and additive fashion.

This animal model will enable investigation into disease etiology and identification of factors that modulate the generation of immune related adverse events. This animal model develops autoimmunity in the heart and pancreas (as well as other organs), which is important because fatal myocarditis (inflammation of the heart) and type I diabetes (autoimmune destruction of the pancreas) are two types of rare but very serious complications associated with combination anti-*CTLA4* and anti-PD-1 therapy in human patients. This model also appears to be able to model other types of autoimmune adverse events (e.g. gastrointestional) that are associated with checkpoint blockade. The transgenic mouse model described can be used as a model of combination anti-*CTLA4* plus anti-PD-1 immune checkpoint blockade (i.e., treatment of monoclonal antibodies targeting T cell costimulatory receptors *CTLA4* and PD-1). Genetic loss of PD-1 and single copy loss of *CTLA4* models this therapy in an analogous scenario in which negative costimulatory activity is reduced and modeled. Thus, this genetic model that modulates *CTLA4* and PD-1 recapitulates the phenomena of immune-related adverse events due to checkpoint blockade therapy. This is particularly notable because there are currently no animal models that faithfully recapitulate the adverse events associated with checkpoint blockade therapy. Combination anti-*CTLA4* and anti-PD-1 checkpoint blockade therapy is currently approved for the treatment of melanoma and renal cell carcinoma (along with over 250 on-going clinical trials).

In addition, this mouse strain background is C57BL6/J, which is notable because of the widespread use of this background for tumor immunology studies and also because this background is normally very difficult to induce autoimmunity in, suggesting that this phenotype satisfies a high biological bar. Also, no exogenous antigens were introduced (e.g. transgenic, viral) and thus the antigens that are being recognized to drive this autoimmunity are self-antigens, as is presumed to be the case in the setting of patients that receive checkpoint blockade therapies. Furthermore, because multiple types of autoimmunity and immune-related disease etiologies arise in this animal model, this enables the investigation of the relationships between these diseases.

Thus, this mouse model can be used to understand how these adverse events are induced as well as to test the efficacy of therapies that aim to mitigate such adverse events and autoimmunity. Such investigation is likely necessary to design next-generation immunotherapies that retain therapeutic efficacy and reduce adverse events, particularly induction of rare, very serious autoimmunity.

### I. Aspects of the Present Invention

A genetic interaction between the T cell negative costimulatory genes *Ctla4* and *Pdcd1* (encoding PD-1) is identified herein. This genetic interaction manifests as conditional haploinsufficiency of *Ctla4* in the context of complete absence of *Pdcd1,* which leads to fatal systemic autoimmunity. From a fundamental perspective, this observation supports a threshold model of T cell activation in which multiple sources of T-cell receptor (TCR) signal perturbation can compound to result in aberrant T cell activation. This indicates that *CTLA4* and PD-1 regulatory signals are functionally integrated and together provide a critical buffering system to restrain T cell activation. At the molecular level, decreases in the combined gene dosage of *Pdcd1* and *Ctla4* may limit the overall ability to attenuate T cell activation in a cell intrinsic manner.

In addition to the significant insights into basic mechanisms of T cell activation, these findings have notable clinical implications in the context of cancer immune checkpoint blockade. Combination anti-*CTLA4* plus anti-PD-1 therapy is effective in multiple tumor types, including advanced melanoma and renal cell carcinoma. Anti*-CTLA4* and anti-PD-1 immune checkpoint blockade are known to utilize distinct cellular mechanisms (Das *et al.,* 2015; Wei *et al.,* 2017). Taken in the context of the present findings, these distinct cellular mechanisms likely interact functionally, which may in part explain the enhanced efficacy of combination therapy versus monotherapies (Curran *et al.,* 2010; Postow *et al.,* 2015; Wolchok *et al.,* 2013). Even more relevant to the present findings, immune checkpoint blockade therapy can induce serious immune-related adverse events as well as *bona fide* autoimmunity, such as myocarditis and type I diabetes, in rare instances. *Ctla4*^{+/}*⁻ Pdcd1*^{*-*/*-*} mice provide a preclinical animal model with which to study autoimmunity induced by loss of *CTLA4* and PD-1 signaling. This is particularly notable given that autoimmunity observed in this model closely reflect the myocarditis and type I diabetes that can arise following combination anti-*CTLA4* plus anti-PD-1 checkpoint blockade therapy in human patients. Further mechanistic understanding provides the potential to distinguish and specifically modulate aspects of the immunological response that mediate efficacy and adverse events of immune checkpoint blockade therapy.

Interestingly, the autoimmunity that develops in *Ctla4*^{+/}*⁻ Pdcd1*^{*-*/*-*} mice recurrently manifests in specific anatomical sites. Why particular tissue sites are more sensitive to autoimmunity induced by loss of PD-1 and *CTLA4* signaling remains a critical open question. It is possible that tissue specific antigens from these sites render them particularly liable to autoimmune recognition in the absence of negative co-stimulation. Alternatively, it is possible that tissue sensitivity is due to functional differences between tissue-specific T_{reg} populations that have been previously observed (Legoux *et al.,* 2015).

Notably, heterozygous germ line loss of function alleles of *CTLA4* lead to immune dysregulation with highly variable clinical presentation (Kuehn *et al.,* 2014; Schubert *et al.,* 2014). This indicates that single copy loss of *CTLA4* in humans is pathogenic and furthermore, is strongly suggestive of genetic interaction with other genetic and/or environmental factors. It is also possible that single copy loss of *CTLA4* in humans or in mice (in the absence of PD-1) lowers the T cell activation threshold to the level at which tonic TCR signaling can reach, and thus stochastic processes may explain the variance in clinical presentation in *CTLA4* deficient humans and mice. An outstanding question is the extent to which other T cell costimulatory molecules or molecules involved in their function genetically interact with *Pdcd1* and *Ctla4.* For example, patients harboring loss of function alleles of *LRBA,* an important regulator of *CTLA4* trafficking, present with similar autoimmune phenotypes as patients harboring loss of function *CTLA4* (Besnard *et al.,* 2018; Hou *et al.,* 2017).

In addition to the key finding of genetic interaction between *CTLA4* and PD-1, the present findings also suggest that simultaneous genetic deficiency of *Ctla4* and *Pdcd1* is embryonic lethal. This is surprising given that αβ T cells do not emerge until after birth (Havran and Allison, 1988). The mechanism through which this occurs remains unclear, however there are two main possibilities, both of which are quite intriguing. The first possibility is that *CTLA4* and PD-1 restrict activation of γδ T cells, which emerge as early as E14 during embryonic development. The second possibility is that *CTLA4* and PD-1 may have as yet unidentified non-immunological functions during development.

In conclusion, the present findings reveal genetic interaction between *Ctla4* and *Pdcd1.* This provides definitive evidence for functional interaction between the regulatory mechanisms of *CTLA4* and PD-1. Furthermore, this provides a robust animal to model immune-related adverse events induced by combination anti-CTLA4 plus anti-PD-1 immune checkpoint blockade therapy.

### II. Examples

The following examples are included to demonstrate preferred embodiments of the invention.

### Materials and Methods

*Mice. Ctla4*^{tm1All} mice (Chambers *et al.,* 1997) were bred to *Pdcd1* knockout mice (*Pdcd1*^{tm1.1Shr}) (Keir *et al.,* 2007), which were purchased from The Jackson Laboratory (021157). *Pdcd1* knockout mice were backcrossed once to C57BL/6J prior to this cross. Resulting F1 *Ctla4*^{+/}*⁻ Pdcd1*^{+/*-*} mice were intercrossed to produce all possible combinations of wild-type and mutant alleles of the two genes. This first breeding scheme specifically utilized F1 mice derived from the cross of *Ctla4*^{+/*-*} (which are wild-type for *Pdcd1*) and *Pdcd1*^{*-*/*-*} mice (which are wild-type for *Ctla4*)*.* This breeding scheme ensures that mutant alleles of *Ctla4* and *Pdcd1* in F1 mice are in *trans,* and thus the recombination frequency can be calculated (FIG. 9A). The genetic distance between *Ctla4* and *Pdcd1* was calculated by assessing the number of recombination and total events in this cross. The observed recombination frequency of 17.03 cM closely aligned with the 16.89 centi-Morgan genetic distance between *Ctla4* and *Pdcd1* reported by the Mouse Phenome Database (MPD, RRID:SCR_003212) (Bogue *et al.,* 2018). This estimated genetic distance was consistent with the observed recombination frequency in this breeding scheme.

To verify findings from the first breeding scheme, a second related breeding scheme was utilized. Importantly, this breeding approach utilized different genotypes, the mutant alleles could either be in *cis* or *trans,* and the approach would generate *Ctla4*^{+/-} *Pdcd1*^{*-*/-} (experimental) and *Ctla4*^{*+*/}*⁺ Pdcd1*^{*-*/*-*} (control littermates) in a 1:1 ratio. This allows for the generation of many more *Ctla4*^{+/}*⁻ Pdcd1*^{*-*/*-*} mice than in the initial breeding approach. Specifically, male *Ctla4*^{+/-} *Pdcd1*^{*-*/*-*} and female *Ctla4*^{*+*/}*⁺ Pdcd1*^{*-*/*-*} were bred. Female *Ctla4*^{+/+} *Pdcd1*^{*-*/*-*} were used to eliminate the possibility that the autoimmunity observed in *Ctla4*^{+/}*⁻ Pdcd1*^{*-*/*-*} might affect fetal-maternal tolerance or the ability to produce viable litters.

For the generation of survival curves, events were defined as either death (i.e. mice found dead) or identification of mice by veterinary staff as requiring euthanasia (e.g. due to lethargy, moribund, dyspnea). For mice identified as requiring euthanasia, the date of death was defined as the day the mouse was flagged by veterinary staff. Animal phenotypes associated with mortality were identified and reported by veterinary staff. Mice utilized for breeding were censored from survival analyses at the time that they were utilized for this purpose.

All mice were housed at The University of Texas MD Anderson Cancer Center South Campus Vivarium, an AAALAC-accredited specific pathogen-free animal facility. All experiments were performed in accordance with The University of Texas MD Anderson Cancer Center Institutional Animal Care and Use Committee (IACUC) guidelines.

*Genotyping.* Genomic DNA was isolated using Direct-to-PCR digest mix and polymerase chain reaction (PCR) based genotyping was performed for *Ctla4* and *Pdcd1* knockout mice. Primers are provided in Table 1. *Ctla4*^{tm1All} mice were genotyped as previously described (Chambers *et al.,* 1997). The expected band sizes for the *Ctla4* wild-type and mutant alleles are ~75 and ~150 bp, respectively. *Pdcd1* knockout mice were genotyped as previously described (Keir *et al.,* 2007). The expected band sizes for the *Pdcd1* wild-type and mutant alleles are 418 and 350 bp, respectively.

**Table 1. Primers for genotyping.**

| | | Sequence | SEQ ID NO: |
|---|---|---|---|
| *CTLA4* primers | | 5' AAACAACCCCAAGCTAACTGCGACAAGG 3' | 1 |
| | | 5' CCAGAACCATGCCCGGATTCTGACTTC 3' | 2 |
| | | 5' CCAAGTGCCCAGCGGGGCTGCTAAA 3' | 3 |
| *Pdcd1* primers | PD1 KO common | 5' CACTATCCCACTGACCCTTCA 3' | 4 |
| | PD1 KO WT | 5' AGAAGGTGAGGGACCTCCAG 3' | 5 |
| | PD1 KO Mut rev | 5' CACAGGGTAGGCATGTAGCA 3' | 6 |

*SNP typing.* Crude genomic DNA lysate was submitted to The University of Texas MD Anderson Cancer Center Laboratory Animal Genetics Services core facility for SNP-typing using a 100-marker panel. For the purpose of determining whether genetic variants associate with autoimmunity, 'unaffected' mice were defined as mice that did not manifest any symptoms or die within 6 months of age and 'affected' mice were defined as class I mice that manifested symptoms and succumbed to disease.

*Pathology analyses.* Animal necropsies were performed by personnel in The University of Texas MD Anderson Cancer Center veterinary medical histology laboratory or in the Allison laboratory. Automated serum chemistry analysis using a Cobas Integra 400Plus (Roche Diagnostics, Risch-Rotkreuz, Switzerland) was performed on a blood sample collected at euthanasia. Formalin-fixed tissues were processed routinely into paraffin blocks, sectioned at 5 microns, and stained with hematoxylin and eosin. Additional sections were used for immunohistochemical (IHC) staining of particular tissues of interest, using an antibody directed against CD3 (ab 16669, Abcam, Cambridge, MA), followed by secondary reagents for chromogenic detection (Bond Polymer Refine Detection system, DS9800, Leica, Buffalo Grove, IL). Stained sections were examined by a veterinary pathologist using a Leica DM2500 microscope with Leica DFC495 camera and Leica Application Suite v4.12 software. Histologic changes were scored using a semi-quantitative scale, with 0= no lesion to 4= severe lesion.

*Flow cytometry.* Single cell suspensions from lymph nodes were prepared by mashing pooled inguinal, axillary, and brachial lymph nodes through a 70um filter using the back of a plastic syringe into RPMI-1640 supplemented with 10% FBS and 1% Penicillin Streptomycin. A 96-well flat bottom plate was coated 200ul per well of 1ug/ml anti-CD3ε and 2ug/ml anti-CD28 in PBS overnight at 4°C the previous night. Cells were then stained with CellTrace Violet Proliferation kit per the manufactures protocol (Invitrogen, C34557). Triplicates of each sample were plated 10⁶ cells/mL per well in 200ul of RPMI-1640 supplemented with 10% FBS, sodium pyruvate, 0.1% b-ME, and P/S and incubated at 37°C for 46 hours. Cells were then transferred to a U-bottom 96-well plate and washed twice with FACS buffer and incubated with 2% of each bovine, murine, rat, hamster, and rabbit serum PBS with 25 mg/mL 2.4G2 antibody at 4°C for 10 min prior to surface staining with an antibody cocktail at 4°C for 30 min in a 50 mL volume. Cells were washed twice with FACS buffer then fixed and permeabilized using the FoxP3 fix and permeabilization kit according to manufacturer's protocol (eBioscience). Cells were subsequently stained with an intracellular antibody cocktail at room temperature for 30 min. Cells were then washed twice with Foxp3 permeabilization buffer, then twice with FACS buffer, and analyzed on a LSRII (BD).

For surface stain (restim) the following antibodies were used: LIVE/DEAD^{™} Fixable Blue Dead Cell Stain L23105 (ThermoFisher); BV786 Hamster Anti-Mouse CD3e (clone 145-2C11, 564379 (BD)); Brilliant Violet 605 anti-mouse TCR β chain Antibody (clone)H57-597, 109241(BioLegend)); Brilliant Violet 650 anti-mouse CD19 Antibody (clone 6D5, 115541 (BioLegend)); FITC Anti-mouse CD4 Antibody (clone RM4.5, 11-0042-82 (ebio)); PE anti-mouse CD152 Antibody (clone UC10-4B9, 106306 (BioLegend)); PE Armenian Hamster IgG Isotype Ctrl Antibody (clone HTK888, 400908 (Biolegend); APC Anti-mouse CD8a Antibody (clone 53-6.7, 17-0081-82 (ebio)); and Alexa Fluor 700 Anti-mouse CD45.2 Antibody, (clone 104, 56-0454-82 (ebio)). For IC stain (restim) the following antibodies were used: BV786 Hamster Anti-Mouse CD3e (clone 145-2C11, 564379 (BD)); Brilliant Violet 605 anti-mouse TCR β chain Antibody (clone H57-597, 109241(BioLegend)); FITC Anti-mouse CD4 Antibody (clone RM4.5, 11-0042-82 (ebio)); PE anti-mouse CD152 (CTLA-4) Antibody (clone UC10-4B9, 106306 (BioLegend)); PE Armenian Hamster IgG Isotype CTLA-4 Ctrl Antibody (clone HTK888, 400908 (BioLegend)); and APC Anti-mouse CD8a Antibody (clone 53-6.7, 17-0081-82 (ebio)).

*Luminex cytokine and chemokine assessment.* Serum was collected from *Ctla4*^{+/}*⁻ Pdcd1*^{*-*/*-*} and control littermate mice (including both *Ctla4*^{+/+} *Pdcd1*^{*-*/*-*} mice and mice competent for both *CTLA4* and PD-1 such as *Ctla4*^{+/}*⁻ Pdcd1*^{*-*/*-*} mice) from both breeding schemes described above. Briefly, blood was collected by terminal cardiac puncture, allowed to coagulate at room temperature, centrifuged at 8,000g for 10 minutes, supernatant serum collected and snap frozen in liquid nitrogen prior to storage at -80 degrees Celsius. Serum levels of antibodies cytokine and chemokine were assessed using the Cytokine & Chemokine 36-plex Mouse ProcartPlex luminex assay (ThermoFisher Scientific) per manufacturer's protocol. All samples were analyzed in parallel in a single batch for each respective analysis. Serum samples were diluted 1:10,000 for analysis of serum antibody levels.

*Reverse phase proteomic array analysis.* Lymph nodes from 16-day-old *Ctla4* knockout and littermate control mice were snap frozen for subsequent analysis. Tissue samples were lysed in 1% Triton X-100, 5 0mMHEPES, pH 7.4, 150 mMNaCl, 1.5 mM MgCl₂, 1 mM EGTA, 100 mM NaF, 10 mM Na pyrophosphate, 1 mM Na₃VO₄, 10% glycerol, with freshly added protease inhibitors (Roche, 05056489001) and phosphatase inhibitors (Roche, 04906837001) and homogenized using a Precellys homogenizer (Bertin Instruments). Samples were diluted in sample buffer (10% glycerol, 2% SDS, 62.5 mM Tris-HCl, BME, pH 6.8) prior to array printing and analysis. Signal was quantified using Array-Pro Analyzer software (MediaCybernetics) and normalized using a "Supercurve fitting" approach developed at MD Anderson Cancer Center for the RPPA Core Facility. Normalized linear values were analyzed in Excel (Microsoft) using two-tailed T-test assuming unequal variance and plotted using Prism 6.0 (GraphPad).

*Nanostring mRNA analysis.* Lymph nodes were dissected from *Ctla4* knockout and littermate control mice. RNA was extracted from lymph nodes using (Qiagen). 100 ng RNA was analyzed using the Mouse Immunology Code set panel on the nCounter platform (Nanostring). Values were normalized on a per sample basis using housekeeping genes within the panel. Heat maps of Nanostring gene expression and RPPA proteomic data were generated in R utilizing a Pearson distance matrix and Ward's minimum variance method. Only differentially expressed genes, defined by a false discovery rate of 5% with a one-way ANOVA comparison between genotypes, were plotted.

*Statistics.* Statistical analyses were performed in Prism 7.0 or 8.0 (GraphPad Software, San Diego, CA), unless otherwise noted. Normalized linear values of RPPA data were analyzed in Excel (Microsoft) using two-tailed T-test assuming unequal variance and plotted.

### Example 1 - Lethal haploinsufficiency of Ctla4 in the genetic absence of Pdcd1

To test whether there is a genetic interaction between *Ctla4* and *Pdcd1, Ctla4* and *Pdcd1* (encoding PD-1) knockout transgenic mice were crossed. Murine *Ctla4* and *Pdcd1* are genetically linked with a genetic distance of 16.89 cM based on estimations from the Mouse Phenome Database (Bogue *et al.,* 2018). A heterozygous intercross-breeding scheme (see Materials and Methods) was used that allowed for the generation of all possible permutations of mutant alleles from a single cross and for estimation of the observed recombination frequency between *Ctla4* and *Pdcd1* at 17.03 cM. Surprisingly, approximately 50% of *Ctla4*^{+/}*⁻ Pdcd1*^{*-*/*-*} spontaneously died within 3 months of age (FIG. 1A). In contrast, related control littermates (e.g. *Ctla4*^{*+*/}*⁺ Pdcd1*^{*-*/*-*} and *Ctla4*^{+/}*⁻ Pdcd1*^{*+*/*-*}) exhibited no overt phenotypes and no deaths were observed in these groups. The lack of an overt phenotype in *Ctla4*^{+/+} *Pdcd1*^{*-*/*-*} mice is consistent with prior observations (Nishimura *et al.,* 1999). In addition, death of all of the *Ctla4*^{-/-} *Pdcd1*^{+/+} mice is consistent with the initial characterization of *CTLA4* deficient mouse strains (Chambers *et al.,* 1997; Tivol *et al.,* 1995; Waterhouse *et al.,* 1995). Interestingly though, monoallelic loss of *Pdcd1* accelerated the fatal lymphoproliferation induced by loss of *CTLA4* (FIG. 1A). This suggests that *Pdcd1* gene dosage modifies the phenotype and lymphoproliferative disease of *CTLA4* deficient mice. These observations contrast the absence of any PD-1 haploinsufficiency in *Ctla4*^{*+*/}*⁺ Pdcd1*^{+/*-*} or *Ctla4*^{+/}*⁻ Pdcd1*^{+/*-*} mice.

Of particular interest was the surprising spontaneous death of *Ctla4*^{+/}*⁻ Pdcd1*^{*-*/*-*} mice as this provides strong evidence of genetic interaction. To further confirm this observation, *Ctla4*^{+/-} *Pdcd1*^{*-*/*-*} and *Ctla4*^{*+*/}*⁺ Pdcd1*^{*-*/*-*} littermate mice were generated using a different breeding scheme (see Materials and Methods). This second approach yielded remarkably similar findings with approximately 50% of *Ctla4*^{+/}*⁻ Pdcd1*^{*-*/*-*} mice spontaneously dying while no deaths were observed in littermate *Ctla4*^{+/+} *Pdcd1*^{*-*/*-*} mice (FIG. 1B). Mice spontaneously died or became moribund between 2-6 months of age, with a variety of observed clinical symptoms (Table 2, see Materials and Methods). The onset of these specific phenotypes is not necessary per say for the fatal autoimmunity observed however, given that of mice with and without prior presentation of these symptoms has similar latency of death (FIG. 9B). Interestingly however, male and female *Ctla4*^{+/}*⁻ Pdcd1*^{*-*/*-*} mice died at significantly different frequencies (FIG. 1C). This indicates that the observed conditional haploinsufficiency of *Ctla4* is sex-dependent, with female mice dying at higher frequency than male mice. This sex imbalance is consistent with the increased overall risk of irAEs in female patients receiving anti-CTLA-4 ICI (Valpione *et al*., 2018). Mortality was preceded by overt, non-specific clinical signs (e.g. reduced weight gain, ataxia, dyspnea) beginning as early as 1 month of age in approximately two-thirds of mice that died. Reflective of the severity of this phenotype the total body weight of Ctla4^{+/-} Pdcd1^{-/-} mice displaying clinical signs was significantly lower than that of Ctla4^{+/-} Pdcd1^{-/-} mice not displaying clinical signs (FIG. 2B).

Together these observations indicate that *Pdcd1* and *Ctla4* exhibit strong genetic interaction. Specifically, there is a dramatic *Ctla4* conditional haploinsufficiency, which manifests only in the context of genetic deletion of *Pdcd1* and leads to spontaneous deaths (FIGS. 1A-B).

**Table 2. Phenotypes associated with mortality of Ctla4^{+/}⁻ Pdcd1^{-/-} mice. The absolute number and relative frequency of phenotypes associated with Ctla4^{+/}⁻ Pdcd1^{-/-} mice that were found dead or identified as requiring euthanasia.**

| | No observed phenotype | Small | Thin/ Emaciated | Hunched | Rough coat | Lethargic | Paresis | Hyperpnea | Ataxia | Abdominal swelling |
|---|---|---|---|---|---|---|---|---|---|---|
| Frequency | 45.45 | 909 | 50 | 45.45 | 13.63 | 31.81 | 4.54 | 13.63 | 9.09 | 4.54 |

Given that only 50% of *Ctla4*^{+/-} *Pdcd1*^{*-*/*-*} mice die, it is likely that additional genetic or environmental factors modulate the penetrance of *Ctla4* conditional haploinsufficiency. Whether subtle genetic differences could underlie this dichotomy was investigated. All tested mice were 97-100% C57BL6/J based on a 100-marker single nucleotide polymorphism typing panel, with no associations between any segregating alleles with phenotypic manifestation were observed (FIG. 7 and FIG. 8).

It was then determined whether *Ctla4*^{+/}*⁻ Pdcd1*^{*-*/*-*} mice that did not spontaneously die and reached the age of the survival plateau (approximately 6 months) harbored subtle autoimmunity, which is biologically relevant but not sufficient to cause death. In contrast, the total body weights of affected *Ctla4*^{+/}*⁻ Pdcd1*^{*-*/*-*} mice were significantly reduced compared to either phenotypically normal *Ctla4*^{+/}*⁻ Pdcd1*^{*-*/*-*} or *Ctla4*^{+/+} *Pdcd1*^{*-*/*-*} mice (FIG. 2A). In contrast, the total body weight of phenotypically normal *Ctla4*^{+/}*⁻ Pdcd1*^{*-*/*-*} and control littermate *Ctla4*^{+/+} *Pdcd1*^{*-*/*-*} mice were not significantly different (FIG. 2B). To further investigate whether biological, basic serum chemistry was performed on samples from aged ([4+] months) unaffected *Ctla4*^{+/-} *Pdcd1*^{*-*/}*⁻,* and *Ctla4*^{+/+} *Pdcd1*^{*-*/*-*} mice. Interestingly, no significant differences between genotypes were observed (FIG. 2B). This suggests that unaffected mice (defined as the absence of phenotypic decline leading to death) do not develop significantly increased autoimmunity, at least detectable by markers of systemic tissue damage (*e.g.*, LDH) or visual inspection. Notably, however, analyses of serum chemistries phenotypically affected *Ctla4*+/*- Pdcd1-*/*-* mice revealed evidence of tissue damage with significantly elevated serum levels of ALT, AST, and LDH as well as decreased glucose levels (Fig. 2C). The findings suggest that tissue destruction in *Ctla4+*/*- Pdcd1-*/*-* mice, and is detectable in peripheral blood.

These data support a model in which environmental factors modulate the penetrance and development of fatal phenotypes in *Ctla4*^{+/}*⁻ Pdcd1*^{*-*/*-*} mice. From a fundamental perspective, this observation supports a threshold model of T cell activation in which multiple sources of TCR signal perturbation are integrated to regulate T cell activation. In the case of *Ctla4*^{+/}*⁻ Pdcd1*^{*-*/*-*} mice, the threshold for activation is significantly lowered such that additional subtle inputs, which are normally buffered, are sufficient to induce aberrant T cell activation and autoimmunity. A prediction of this model is that T cells derived from *Ctla4*^{+/}*⁻ Pdcd1*^{*-*/*-*} mice have decreased levels of CTLA-4 compared to T cells derived from *Ctla4*^{*+*/}*⁺ Pdcd1*^{*-*/*-*} mice. To confirm that single copy loss of *Ctla4* leads to a decrease in available CTLA-4 protein, we assessed total CTLA-4 protein expression in activated T cells from *Ctla4*^{+/}*⁻ Pdcd1*^{*-*/*-*} and littermate control *Ctla4*^{*+*/}*⁺ Pdcd1*^{*-*/*-*} mice. Notably, flow cytometry analysis of in vitro stimulated T cells are suggestive of lower levels of CTLA-4 protein in T cells derived from *Ctla4*^{+/}*⁻ Pdcd1*^{*-*/*-*} mice compared to T cells derived from *Ctla4*^{+/+} *Pdcd1*^{*-*/*-*} mice (FIG. 2D).

Taken in the context of prior findings that *Ctla4*^{+/*-*} mice do not display any haploinsufficiency at either the organismal or cellular level, these findings indicate that PD-1 negative co-stimulation is sufficient to functionally buffer mono-allelic loss of *Ctla4.* Consistent with this notion, few differences were observed in transcriptional and proteomic analyses of lymph nodes from *Ctla4*^{*+*/*+*} and *Ctla4*^{+/*-*} mice where dramatic changes were observed in *Ctla4*^{*-*/*-*} mice (FIGS. 5 & 6).

Interestingly, these results contrast the findings from mass cytometry profiling of similar tissues from *Ctla4*^{*+*/*+*} and *Ctla4*^{+/-} mice (with no perturbation in *Pdcd1*)*,* in which no differences in cellular phenotype or frequency were detected. This suggests that at least in homogeneous inbred murine strains, additional regulatory molecular mechanisms can buffer against perturbations in signaling caused by single copy loss of *Ctla4.* However, in the context of additional perturbations, such as genetic loss of PD-1, functional defects in T cell regulation due to mono-allelic *Ctla4* can manifest due to a loss of buffering capacity.

### Example 2 - Ctla4^{+/-} Pdcd1^{-/-} mice develop multi-tissue autoimmunity

It was next sought to understand the cause of death of *Ctla4+*/*- Pdcd1-*/*-* mice and investigate whether specific tissues were affected. In addition, it was sought to understand whether particular cell types mediated disease etiology. To address these questions, 42 tissues from *Ctla4*^{*+*/}*⁺ Pdcd1*^{*-*/*-*} and *Ctla4*^{+/}*⁻ Pdcd1*^{*-*/*-*} mice were histologically analyzed (see Materials and Methods). Inflammation was observed in multiple tissues including heart, pancreas, lung, liver, and gastrointestional tract. Specific pathologic observations include lymphocytic myocarditis, endarteritis, pulmonary vasculitis, and lymphocytic pancreatitis. Of the most dramatic histological findings, significant immune infiltrate was observed in heart and pancreatic tissues of *Ctla4*+/*- Pdcd1-*/*-* mice (FIG. 3A-D; FIG. 10 and FIG. 11A). Additional pathologic observations more minor in nature, but also notable, include lymphoid infiltrates in the salivary gland, lacrimal gland, harderian gland, and kidney. Other minor observations include hepatic degeneration/necrosis, lymphocytic gastritis, and synovitis although the degree to which these findings associate with genotype remain not fully determined. Interestingly, the more significant histological findings were observed in non-lymphoid peripheral tissues rather than lymphoid organs such as the spleen or lymph nodes. This suggests that peripheral immunological tolerance is specifically breached in *Ctla4*^{+/}*⁻ Pdcd1*^{*-*/*-*} mice.

To better interrogate nature of the myocardial infiltrates, detailed H&E histological analyses of a larger cohort of mice as well as immunohistochemical staining of similar tissue samples for T cells (utilizing CD3 as a pan-T cell marker) was performed. Myocarditis consisted of significant T cell infiltration in *Ctla4*^{+/}*⁻ Pdcd1*^{*-*/*-*} mice. Histological analyses also were suggestive of infiltration of other immune populations such as macrophages. These data suggest that the myocarditis seen in the Ctla4+/- Pdcd1*-*/*-* mice is histologically similar to patients with ICI-associated myocarditis. The results of a range of histological analyses are summarized below in Tables 3-4.

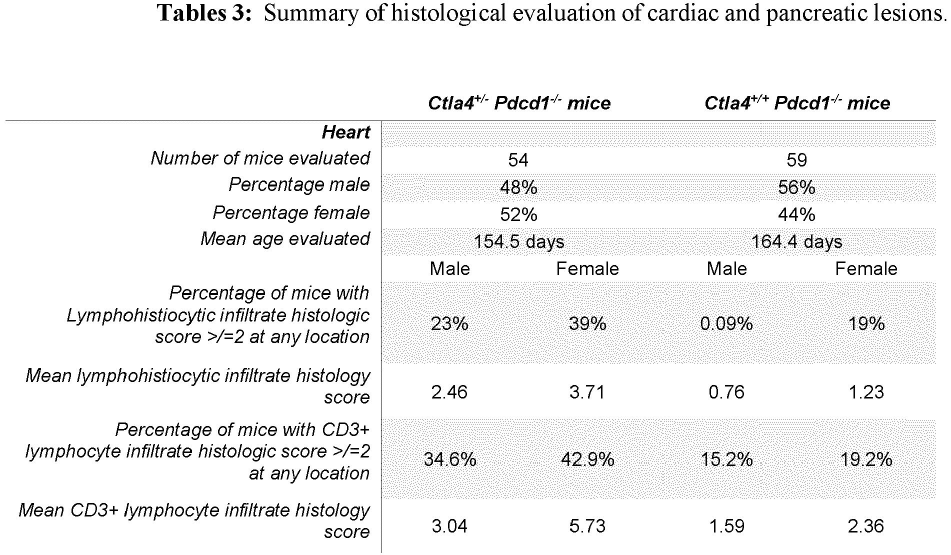

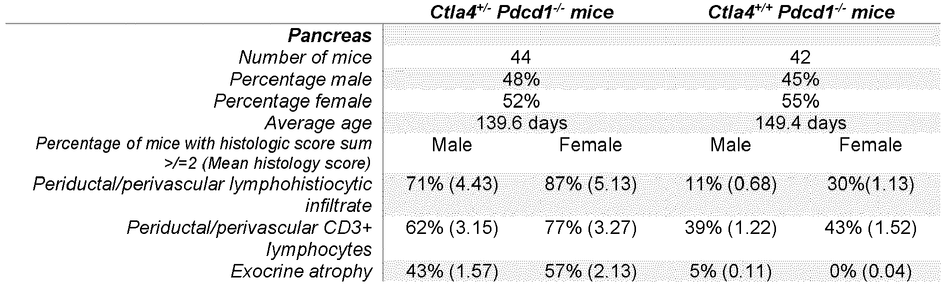

**Table 4: Histological analyses of Ctla4^{+/}⁻ Pdcd1^{-/-} and littermate Ctla4^{+/+} Pdcd1^{-/-} control mice. Semi-quantitative histologic scores across a broad range of tissues. Mouse characteristics including age, sex, symptoms observed, and genotype are denoted. Lesions considered to be incidental or mouse strain-related are not reported in the table. Distribution of lesions by genotype, age, sex, with threshold of scores reported. Not all anatomic structures were available for evaluation in the examined sections.**

| **First group of mice on which complete necropsies performed** | ***Ctla4*^{+/}*⁻ Pdcd1*^{*-*/*-*}** | | | | ***Ctla4*^{+/+} *Pdcd1*^{*-*/*-*}** | | | |
|---|---|---|---|---|---|---|---|---|
| **Age** | <110 Days | | >110 Days | | <110 Days | | >110 Days | |
| **Sex** | Male | Female | Male | Female | Male | Female | Male | Female |
| **Cardiovascular** | | | | | | | | |
| Ventricular endocarditis, mononuclear, score>/=1 | **2/3** | 0/2 | 0/0 | 0/1 | 0/4 | 0/2 | 0/0 | **1/1** |
| Ventricular myocarditis, mononuclear, score>/=1 | **2/3** | **1/2** | 0/0 | 0/1 | 0/4 | 0/2 | 0/0 | **1/1** |
| Ventricular epicarditis, mononuclear, score>/=1 | **1/3** | 0/2 | 0/0 | 0/1 | 0/4 | 0/2 | 0/0 | 0/1 |
| Atrial myocarditis, score>/=1 | **2/3** | 0/1 | 0/0 | 0/1 | 0/4 | 2/2 | 0/0 | 0/1 |
| Aortic arteritis, score>/=1 | **1/2** | 0/0 | 0/0 | 0/0 | 0/4 | 0/2 | 0/0 | 0/1 |

| **Pancreas** | | | | | | | | |
|---|---|---|---|---|---|---|---|---|
| Insulitis, lymphoid or mononuclear, score >/=1 | **1/3** | 0/2 | 0/0 | 0/1 | 0/4 | 0/2 | 0/0 | 0/1 |
| Exocrine pancreatitis, periductal or | **1/3** | **2/2** | 0/0 | **1/1** | 0/4 | **1/2** | 0/0 | 0/1 |

| **First group of mice on which complete necropsies performed** | ***Ctla4*^{+/}*⁻ Pdcd1*^{*-*/*-*}** | | | | ***Ctla4*^{+/+} *Pdcd1*^{*-*/*-*}** | | | |
|---|---|---|---|---|---|---|---|---|
| parenchymal, lymphoid or mononuclear, score>/=1 | | | | | | | | |
| Loss of exocrine parenchyma, score>/=2 | **1/3** | **2/2** | 0/0 | **1/1** | 0/4 | **1/2** | 0/0 | 0/1 |

| **Pulmonary** | | | | | | | | |
|---|---|---|---|---|---|---|---|---|
| Vasculitis and perivasculitis, chronic, multifocal to coalescing, score>/=2 | **2/3** | 0/2 | 0/0 | 0/1 | 0/4 | /2 | 0/0 | 1/1 |

| **Renal** | | | | | | | | |
|---|---|---|---|---|---|---|---|---|
| Lymphoid or mononuclear infiltrate, interstitial, hilar, or capsular, score>/=2 | 1/3 | 0/2 | 0/0 | 0/1 | 3/4 | 0/2 | 0/0 | 1/1 |
| Cortical tubular basophilia, consistent with chronic progressive nephropathy, score>/=2 | 0/3 | 0/2 | 0/0 | 0/1 | 1/4 | 1/2 | 0/0 | 0/1 |

| **Lymphoid Organs** | | | | | | | | |
|---|---|---|---|---|---|---|---|---|
| Cyst, thymus | 0/3 | 0/0 | 0/0 | 0/1 | **2/4** | 0/2 | 0/0 | **1/1** |
| | | | | | | | | |
| | | | | | | | | |

| **Hepatic** | | | | | | | | |
|---|---|---|---|---|---|---|---|---|
| Portal mononuclear infiltrate, scores>/=1 | 1/3 | 2/2 | 0/0 | 0/1 | 2/4 | 1/2 | 0/0 | 1/1 |
| Cytoplasmic alteration, eosinophilic, hepatocytes, scores >/=2 | 0/3 | 1/2 | 0/0 | 1/1 | 0/4 | 1/2 | 0/0 | 0/1 |
| Hepatitis, subacute, focal or multifocal, scores >/=2 | 2/3 | 0/2 | 0/0 | 0/1 | 0/4 | 2/2 | 0/0 | 1/1 |
| | | | | | | | | |

Severe atrophy of adipose tissue associated with a wide range of anatomical sites including lung and subcutaneous (skin) was also observed (FIG. 10). It remains unclear whether this phenotype is secondary to the loss of pancreatic exocrine function or a direct effect due to autoimmune recognition of adipose tissue. In addition, atrophy of female reproductive organs was also observed.

These findings are further notable given that the C57BL6/J inbred strain of mice is highly resistant to the development of autoimmunity. For example, consistent with the findings here, mice deficient for PD-1 develop more severe autoimmunity on a Balb/c background compared to a C57BL6/J background (Nishimura *et al.,* 1999; Nishimura *et al.,* 2001). It is also important to note that the autoimmunity that develops due to genetic loss of PD-1 in aged mice is primarily mediated by auto-antibodies. However, antibody levels were not elevated in Ctla4^{+/-} Pdcd1^{-/-} mice (FIG. 11B). This contrasts the lymphocytic infiltration of peripheral tissues observed in *Ctla4*^{+/-} *Pdcd1^{-l-}* mice.

Of note, these findings bear striking resemblance to the autoimmunity and other immune related adverse events (irAEs) associated with combination anti-CTLA4 plus anti-PD-1 immune checkpoint blockade therapy (e.g., ipilimumab plus nivolumab) (Sznol *et al.,* 2017). In particular, the severe autoimmunity observed in pancreatic and cardiac tissue observed in *Ctla4*^{+/-} *Pdcd1^{-l-}* mice appears to be analogous to the fulminant myocarditis and insulin-dependent diabetes mellitus that are rare but very serious adverse events associated with therapeutic blockade of CTLA-4 and PD-1 (Barroso- Sousa *et al.,* 2018; Johnson *et al.,* 2016; Moslehi *et al.,* 2018). Supportive of the notion that *Ctla4*^{+/-} *Pdcd1^{-l-}* mice closely recapitulates this biology, therapy associated myocarditis and diabetes appear to be directly T cell mediated.

It was then sought to gain insight into the antigen specificity of the T cells that underlies the systemic autoimmunity induced by conditional haploinsufficiency of *Ctla4,* and in particular, whether particular tissue antigens were being recurrently recognized. To explore this possibility, TCR sequencing was performed on lymph node, heart, and pancreatic tissues from *Ctla4*^{+/+} *Pdcd1^{-l-}* and *Ctla4*^{+/-} *Pdcd1^{-l-}* mice (see Materials and Methods). Interestingly, no significant changes in T cell clonality were observed between *Ctla4*^{+/+} *Pdcd1^{-l-}* and *Ctla4*^{+/-} *Pdcd1^{-l-}* mice (FIG. 4). T cell clonality was increased in both strains compared to wild-type or *Ctla4*^{+/-} mice previously characterized. This suggests that loss of PD-1 is sufficient to induce T cell proliferation and expansion of clonotypes, but the pathogenic activity of these clones is limited by additional mechanisms. This is consistent with the absence or long-latency of autoimmune phenotypes in *Pdcd1^{-l-}* mice. However, in this context, single-copy loss of *Ctla4* appears sufficient to remove additional regulatory constraints, which allows for the manifestation of pathogenic activity by expanded T cell clones.

To assess whether autoimmunity due to conditional haploinsufficiency of *Ctla4* in the absence of PD-1 arises solely due to defects in peripheral tolerance, or also defects in central tolerance, thymic development in *Ctla4*^{+/-} *Pdcd1*^{*-*/*-*} and littermate *Ctla4*^{+/+} *Pdcd1^{-l-}* mice were characterized. Mono-allelic loss of *Ctla4* did not affect thymocyte composition, consistent with prior reports that CTLA-4 does not play a critical role during thymic development (Chambers *et al.,* 1997; Wei *et al.,* 2019). Consistent with these observations in lymph node derived T cells, thymic-derived Tregs (newly generated and recirculating) derived from *Ctla4*^{+/-} *Pdcd1^{-l-}* mice expressed decreased CTLA-4 protein. Together these data indicate that *Ctla4* haploinsuffiency leads to a defect in peripheral tolerance rather than a defect in central tolerance.

Finally, the molecular basis of the genetic interaction between *Ctla4 and Pdcd1* was investigated. Single copy loss of *Ctla4* was hypothesized to lead to subtle changes in signaling and transcriptional outputs that in the context of an otherwise wild-type condition, do not modulate T cell activity or phenotype due to robust buffering within T cell activation signaling pathways. However, in the additional absence of PD-1, or perhaps other negative costimulatory molecules, subtle molecular defects due to *Ctla4* haploinsufficiency can manifest overtly. To explore this possibility, we utilized reverse phase proteomic analysis (RPPA) to probe the expression of 238 protein targets in lymph node tissue derived from wild-type, heterozygous, and homozygous *Ctla4* knockout mice. This RPPA panel included an array of signaling molecules and phosphorylated epitopes, and thus is well suited to detect changes in canonical signaling pathways.

As expected, proteins associated with proliferation pathways were highly upregulated in *Ctla4*^{-/-} mice compared to littermate controls, consistent with the lymphoproliferative phenotype of *Ctla4* knockout mice. This included significant increases in CDK1, p-Rb, p-S6, p-CHK1, and p-STAT3, accompanied by down-regulation of p21 (FIG. 5F). Most notably, principal component analysis (PCA) and unsupervised hierarchical clustering suggest that the proteomic profiles of *Ctla4*^{+/-} and *Ctla4*^{+/+} mice are distinct. The difference between these groups is largely driven by the down-regulation of multiple proteins in *Ctla4*^{+/-} mice, such as p21, DUSP4, and B7-H3. Likewise, gene expression analyses detected differences between *Ctla4*^{+/-} and *Ctla4*^{*+*/*+*} mice, albeit to a lesser degree, as well as dramatic transcriptional changes in *Ctla4*^{+/-} mice. Although the observed differences in proteomic and transcriptional profiles may reflect changes in cell intrinsic signaling as well as changes in relative cellular composition given that whole lymph node tissue was analyzed, these findings nonetheless indicate that single copy loss of *Ctla4* leads to a subtle molecular haploinsufficiency phenotype. These subtle changes at the proteomic level (FIG. 5) are appear insufficient to modulate immunological responses however, consistent with the absence of such differences at the transcriptional level (FIG. 6). Together these data indicate that PD-1 negative costimulation is sufficient to functionally buffer mono-allelic loss of *Ctla4* in mice. This functional buffer is lost in *Ctla4*^{+/-} *Pdcd1^{-l-}* mice, which thus allows the development and onset of spontaneous autoimmunity.

### REFERENCES

Barroso-Sousa, R., Barry, W.T., Garrido-Castro, A.C., Hodi, F.S., Min, L., Krop, I.E., and Tolaney, S.M. (2018). Incidence of Endocrine Dysfunction Following the Use of Different Immune Checkpoint Inhibitor Regimens: A Systematic Review and Meta-analysis. JAMA Oncol 4, 173-182.
Besnard, C., Levy, E., Aladjidi, N., Stolzenberg, M.C., Magerus-Chatinet, A., Alibeu, O., Nitschke, P., Blanche, S., Hermine, O., Jeziorski, E., et al. (2018). Pediatric-onset Evans syndrome: Heterogeneous presentation and high frequency of monogenic disorders including LRBA and CTLA4 mutations. Clin Immunol.
Bogue, M.A., Grubb, S.C., Walton, D.O., Philip, V.M., Kolishovski, G., Steams, T., Dunn, M.H., Skelly, D.A., Kadakkuzha, B., TeHennepe, G., et al. (2018). Mouse Phenome Database: an integrative database and analysis suite for curated empirical phenotype data from laboratory mice. Nucleic Acids Res 46, D843-D850.
Chambers, C.A., Cado, D., Truong, T., and Allison, J.P. (1997). Thymocyte development is normal in CTLA4-deficient mice. Proceedings of the National Academy of Sciences of the United States of America 94, 9296-9301.
Chemnitz, J.M., Parry, R.V., Nichols, K.E., June, C.H., and Riley, J.L. (2004). SHP-1 and SHP-2 associate with immunoreceptor tyrosine-based switch motif of programmed death 1 upon primary human T cell stimulation, but only receptor ligation prevents T cell activation. Journal of immunology 173, 945-954.
Curran, M.A., Montalvo, W., Yagita, H., and Allison, J.P. (2010). PD-1 and CTLA4 combination blockade expands infiltrating T cells and reduces regulatory T and myeloid cells within B16 melanoma tumors. Proceedings of the National Academy of Sciences of the United States of America 107, 4275- 4280.
Das, R., Verma, R., Sznol, M., Boddupalli, C.S., Gettinger, S.N., Kluger, H., Callahan, M., Wolchok, J.D., Halaban, R., Dhodapkar, M.V., and Dhodapkar, K.M. (2015). Combination therapy with anti- CTLA4 and anti-PD-1 leads to distinct immunologic changes in vivo. Journal of immunology 194, 950-959.
Havran, W.L., and Allison, J.P. (1988). Developmentally ordered appearance of thymocytes expressing different T-cell antigen receptors. Nature 335, 443-445.
Hou, T.Z., Verma, N., Wanders, J., Kennedy, A., Soskic, B., Janman, D., Halliday, N., Rowshanravan, B., Worth, A., Qasim, W., et al. (2017). Identifying functional defects in patients with immune dysregulation due to LRBA and CTLA4 mutations. Blood 129, 1458-1468.
Hui, E., Cheung, J., Zhu, J., Su, X., Taylor, M.J., Wallweber, H.A., Sasmal, D.K., Huang, J., Kim, J.M., Mellman, I., and Vale, R.D. (2017). T cell costimulatory receptor CD28 is a primary target for PD-1-mediated inhibition. Science 355, 1428-1433.
Johnson, D.B., Balko, J.M., Compton, M.L., Chalkias, S., Gorham, J., Xu, Y., Hicks, M., Puzanov, I., Alexander, M.R., Bloomer, T.L., et al. (2016). Fulminant Myocarditis with Combination Immune Checkpoint Blockade. The New England journal of medicine 375, 1749-1755.
Kamphorst, A.O., Wieland, A., Nasti, T., Yang, S., Zhang, R., Barber, D.L., Konieczny, B.T., Daugherty, C.Z., Koenig, L., Yu, K., et al. (2017). Rescue of exhausted CD8 T cells by PD-1-targeted therapies is CD28-dependent. Science 355, 1423-1427.
Keir, M.E., Freeman, G.J., and Sharpe, A.H. (2007). PD-1 regulates self-reactive CD8+ T cell responses to antigen in lymph nodes and tissues. Journal of immunology 179, 5064-5070.
Kroner, A., Mehling, M., Hemmer, B., Rieckmann, P., Toyka, K.V., Maurer, M., and Wiendl, H. (2005). A PD-1 polymorphism is associated with disease progression in multiple sclerosis. Ann Neurol 58, 50- 57.
Krummel, M.F., and Allison, J.P. (1996). CTLA4 engagement inhibits IL-2 accumulation and cell cycle progression upon activation of resting T cells. The Journal of experimental medicine 183, 2533- 2540.
Kuehn, H.S., Ouyang, W., Lo, B., Deenick, E.K., Niemela, J.E., Avery, D.T., Schickel, J.N., Tran, D.Q., Stoddard, J., Zhang, Y., et al. (2014). Immune dysregulation in human subjects with heterozygous germline mutations in CTLA4. Science 345, 1623-1627.
Legoux, F.P., Lim, J.B., Cauley, A.W., Dikiy, S., Ertelt, J., Mariani, T.J., Sparwasser, T., Way, S.S., and Moon, J.J. (2015). CD4+ T Cell Tolerance to Tissue-Restricted Self Antigens Is Mediated by Antigen-Specific Regulatory T Cells Rather Than Deletion. Immunity 43, 896-908.
Lek, M., Karczewski, K.J., Minikel, E.V., Samocha, K.E., Banks, E., Fennell, T., O'Donnell-Luria, A.H., Ware, J.S., Hill, A.J., Cummings, B.B., et al. (2016). Analysis of protein-coding genetic variation in 60,706 humans. Nature 536, 285-291.
Moslehi, J.J., Salem, J.E., Sosman, J.A., Lebrun-Vignes, B., and Johnson, D.B. (2018). Increased reporting of fatal immune checkpoint inhibitor-associated myocarditis. Lancet 391, 933.
Nishimura, H., Nose, M., Hiai, H., Minato, N., and Honjo, T. (1999). Development of lupus-like autoimmune diseases by disruption of the PD-1 gene encoding an ITIM motif-carrying immunoreceptor. Immunity 11, 141-151.
Nishimura, H., Okazaki, T., Tanaka, Y., Nakatani, K., Hara, M., Matsumori, A., Sasayama, S., Mizoguchi, A., Hiai, H., Minato, N., and Honjo, T. (2001). Autoimmune dilated cardiomyopathy in PD- 1 receptor-deficient mice. Science 291, 319-322.
Parry, R.V., Chemnitz, J.M., Frauwirth, K.A., Lanfranco, A.R., Braunstein, I., Kobayashi, S.V., Linsley, P.S., Thompson, C.B., and Riley, J.L. (2005). CTLA4 and PD-1 receptors inhibit T-cell activation by distinct mechanisms. Molecular and cellular biology 25, 9543-9553.
Postow, M.A., Chesney, J., Pavlick, A.C., Robert, C., Grossmann, K., McDermott, D., Linette, G.P., Meyer, N., Giguere, J.K., Agarwala, S.S., et al. (2015). Nivolumab and ipilimumab versus ipilimumab in untreated melanoma. The New England journal of medicine 372, 2006-2017.
Prokunina, L., Castillejo-Lopez, C., Oberg, F., Gunnarsson, I., Berg, L., Magnusson, V., Brookes, A.J., Tentler, D., Kristjansdottir, H., Grondal, G., et al. (2002). A regulatory polymorphism in PDCD1 is associated with susceptibility to systemic lupus erythematosus in humans. Nature genetics 32, 666- 669.
Rau, H., Braun, J., Donner, H., Seissler, J., Siegmund, T., Usadel, K.H., and Badenhoop, K. (2001). The codon 17 polymorphism of the CTLA4 gene in type 2 diabetes mellitus. J Clin Endocrinol Metab 86, 653-655.
Schubert, D., Bode, C., Kenefeck, R., Hou, T.Z., Wing, J.B., Kennedy, A., Bulashevska, A., Petersen, B.S., Schaffer, A.A., Gaining, B.A., et al. (2014). Autosomal dominant immune dysregulation syndrome in humans with CTLA4 mutations. Nature medicine 20, 1410-1416.
Sznol, M., Ferrucci, P.F., Hogg, D., Atkins, M.B., Wolter, P., Guidoboni, M., Lebbe, C., Kirkwood, J.M., Schachter, J., Daniels, G.A., et al. (2017). Pooled Analysis Safety Profile of Nivolumab and Ipilimumab Combination Therapy in Patients With Advanced Melanoma. Journal of clinical oncology: official journal of the American Society of Clinical Oncology 35, 3815-3822.
Tivol, E.A., Borriello, F., Schweitzer, A.N., Lynch, W.P., Bluestone, J.A., and Sharpe, A.H. (1995). Loss of CTLA4 leads to massive lymphoproliferation and fatal multi organ tissue destruction, revealing a critical negative regulatory role of CTLA4. Immunity 3, 541-547.
Vaidya, B., Imrie, H., Perros, P., Dickinson, J., McCarthy, M.I., Kendall-Taylor, P., and Pearce, S.H. (1999). Cytotoxic T lymphocyte antigen-4 (CTLA4) gene polymorphism confers susceptibility to thyroid associated orbitopathy. Lancet 354, 743-744.
Walunas, T.L., Bakker, C.Y., and Bluestone, J.A. (1996). CTLA4 ligation blocks CD28-dependent T cell activation. The Journal of experimental medicine 183, 2541-2550.
Waterhouse, P., Penninger, J.M., Timms, E., Wakeham, A., Shahinian, A., Lee, K.P., Thompson, C.B., Griesser, H., and Mak, T.W. (1995). Lymphoproliferative disorders with early lethality in mice deficient in Ctla4. Science 270, 985-988.
Wei, S.C., Levine, J.H., Cogdill, A.P., Zhao, Y., Anang, N.A.S., Andrews, M.C., Sharma, P., Wang, J., Wargo, J.A., Pe'er, D., and Allison, J.P. (2017). Distinct Cellular Mechanisms Underlie Anti-CTLA4 and Anti-PD-1 Checkpoint Blockade. Cell 170, 1120-1133 e1117.
Wei, S.C., Duffy, C.R., and Allison, J.P. (2018). Fundamental Mechanisms of Immune Checkpoint Blockade Therapy. Cancer Discovery 8, 1-18.
Wolchok, J.D., Kluger, H., Callahan, M.K., Postow, M.A., Rizvi, N.A., Lesokhin, A.M., Segal, N.H., Ariyan, C.E., Gordon, R.A., Reed, K., et al. (2013). Nivolumab plus ipilimumab in advanced melanoma. The New England journal of medicine 369, 122-133.
Zalloua, P.A., Abchee, A., Shbaklo, H., Zreik, T.G., Terwedow, H., Halaby, G., and Azar, S.T. (2004). Patients with early onset of type 1 diabetes have significantly higher GG genotype at position 49 of the CTLA4 gene. Hum Immunol 65, 719-724.

## Claims

1. A mouse whose genome comprises: (i) a heterozygous loss-of-function allele of a *Ctla4* gene and (ii) a homozygous loss-of-function allele of a *Pdcd1* gene.

2. The mouse of claim 1, wherein the mouse has a C57BL/6J genetic background.

3. The mouse of claims 1 or 2, wherein the mouse is a female mouse or a male mouse.

4. The mouse of any one of claims 1 to 3, wherein the heterozygous loss-of-function allele of a *Ctla4* gene is further defined as a heterozygous insertion of a neomycin resistance cassette into exon 3 of the *Ctla4* gene.

5. The mouse of any one of claims 1 to 4, wherein the homozygous loss-of-function allele of the *Pdcd1* gene is further defined as a homozygous deletion of exons 2 and 3 of the *Pdcd1* gene.

6. The mouse of any one of claims 1 to 5, wherein the mouse suffers from autoimmunity.

7. The mouse of claim 6, wherein the autoimmunity is cardiac autoimmunity or pancreatic autoimmunity, preferably wherein the cardiac autoimmunity is myocarditis such as fulminant myocarditis, and/or preferably wherein the pancreatic autoimmunity is insulin-dependent diabetes mellitus, or comprises pancreatic exocrine destruction or pancreatic islet destruction.

8. The mouse of claim 6, wherein the autoimmunity is lymphocytic myocarditis, endarteritis, pancreatic exocrine destruction, pulmonary vasculitis, adipose tissue atrophy, hepatic inflammation, atrophy of female reproductive organs, gastrointestinal tract inflammation, synovitis, or lymphocytic infiltration of the kidney, salivary gland, lacrimal gland, or stomach.

9. A cell isolated from a mouse of any one of claims 1 to 8.

10. The cell of claim 9, wherein the cell is an immune cell, preferably a T cell.

11. A method for screening at least one candidate agent in the mouse according to any one of claims 1 to 8, comprising administering one or more candidate agent to the mouse, wherein the at least one candidate agent is preferably screened for efficacy.

12. The method of claim 11, further comprising
(I) screening the at least one candidate agent in a mouse comprising (i) a homozygous wild-type *Ctla4* gene and (ii) a homozygous loss-of-function allele of a *Pdcd1* gene;
(II) screening the at least one candidate agent in a mouse comprising (i) a homozygous wild-type *Ctla4* gene and (ii) a homozygous wild-type *Pdcd1* gene;
(III) screening the at least one candidate agent in a mouse comprising (i) a homozygous wild-type *Ctla4* gene and (ii) a heterozygous loss-of-function allele of a *Pdcd1* gene;
(IV) screening the at least one candidate agent in a mouse comprising (i) a heterozygous loss-of-function allele of a *Ctla4* gene and (ii) a homozygous wild-type *Pdcd1* gene; or
(V) screening the at least one candidate agent in a mouse comprising (i) a heterozygous loss-of-function allele of a *Ctla4* gene and (ii) a heterozygous loss-of-function of a *Pdcd1* gene.

13. The method of claims 11 or 12, wherein the at least one candidate agent is screened for
(I) its ability to accelerate the development of an immune-related adverse event or immune-related condition;
(II) its ability to worsen the severity of an immune-related adverse event or immune-related condition;
(III) its ability to increase the penetrance of an immune-related adverse event or immune-related condition in a population of the mice; or
(IV) its ability to mitigate an immune-related adverse event or immune-related condition, wherein mitigating an immune-related adverse event or immune-related condition may be further defined as preventing the development of the immune-related adverse event or immune-related condition, or as decreasing the severity of the immune-related adverse event or immune-related condition.

14. The method of any one of claims 11 to 13, wherein the candidate agent is an anti-cancer therapy; a pathogen, stress, an injury, and/or a diet; a syngeneic tumor cell; or a CTLA-4 immunoglobulin fusion protein, a steroid, an agent that depletes a specific population of immune cells, a cytokine modulating agent, or an immunosuppressive agent.

15. The method of claims 13 or 14, wherein
(I) the immune-related adverse event is an autoimmunity;;
(II) the immune-related condition is an autoimmune condition;
(III) the immune-related adverse event or the immune-related condition is chronic or acute; or
(IV) the immune-related adverse event or immune related condition is
(i) inflammation that may be acute or chronic,
(ii) an autoimmunity that represents an autoimmunity induced by a checkpoint blockade therapy in humans or represents an immune-related adverse event in humans;
(iii) lymphocytic myocarditis, endarteritis, pancreatic exocrine destruction, pulmonary vasculitis, adipose tissue atrophy, hepatic inflammation, atrophy of female reproductive organs, gastrointestinal tract inflammation, synovitis, or lymphocytic infiltration of the kidney, salivary gland, lacrimal gland, or stomach; or
(iv) cardiac autoimmunity or pancreatic autoimmunity, preferably wherein the cardiac autoimmunity is myocarditis such as fulminant myocarditis, or preferably wherein the pancreatic autoimmunity is insulin-dependent diabetes mellitus or comprises pancreatic exocrine destruction or pancreatic islet destruction.

## Patentansprüche

1. Maus, deren Genom umfasst: (i) ein heterozygotes Loss-of-Function-Allel eines *Ctla4*-Gens und (ii) ein homozygotes Loss-of-Function-Allel eines *Pdcd1*-Gens.

2. Maus nach Anspruch 1, wobei die Maus einen C57BL/6J genetischen Hintergrund hat.

3. Maus nach Anspruch 1 oder 2, wobei die Maus eine weibliche Maus oder eine männliche Maus ist.

4. Maus nach einem der Ansprüche 1 bis 3, wobei das heterozygote Loss-of-Function-Allel eines *Ctla4*-Gens des Weiteren als heterozygote Insertion einer Neomycin-Resistenz-Kassette in Exon 3 des *Ctla4*-Gens definiert ist.

5. Maus nach einem der Ansprüche 1 bis 4, wobei das homozygote Loss-of-Function-Allel des *Pdcd1-Gens* des Weiteren als homozygote Deletion der Exons 2 und 3 des *Pdcd1-Gens* definiert ist.

6. Maus nach einem der Ansprüche 1 bis 5, wobei die Maus an Autoimmunität leidet.

7. Maus nach Anspruch 6, wobei die Autoimmunität kardiale Autoimmunität oder Pankreas-Autoimmunität ist, bevorzugt wobei die kardiale Autoimmunität Myokarditis wie fulminante Myokarditis ist, und/oder bevorzugt wobei die Pankreas-Autoimmunität Insulin-abhängiger Diabetes mellitus ist oder exokrine Pankreas-Destruktion oder Pankreasinselzellen-Destruktion umfasst.

8. Maus nach Anspruch 6, wobei die Autoimmunität lymphozytäre Myokarditis, Endarteritis, exokrine Pankreas-Destruktion, pulmonale Vaskulitis, Fettgewebe-Atrophie, hepatische Entzündung, Atrophie der weiblichen Fortpflanzungsorgane, Entzündung des Gastrointestinaltrakts, Synovitis oder lymphozytäre Infiltration der Niere, der Speicheldrüse, der Tränendrüse oder des Magens ist.

9. Zelle, die aus einer Maus nach einem der Ansprüche 1 bis 8 isoliert ist.

10. Zelle nach Anspruch 9, wobei die Zelle eine Immunzelle, bevorzugt eine T-Zelle, ist.

11. Verfahren zum Screenen mindestens eines Kandidaten-Agens in der Maus nach einem der Ansprüche 1 bis 8, umfassend das Verabreichen eines oder mehrerer Kandidatenagenzien an die Maus, wobei das mindestens eine Kandidaten-Agens bevorzugt auf Wirksamkeit gescreent wird.

12. Verfahren nach Anspruch 11, des Weiteren umfassend:
(I) Screenen des mindestens einen Kandidaten-Agens in einer Maus, umfassend (i) ein homozygotes Wildtyp-*Ctla4*-Gen und (ii) ein homozygotes Loss-of-Function-Allel eines *Pdcd1-*Gens;
(II) Screenen des mindestens einen Kandidaten-Agens in einer Maus, umfassend (i) ein homozygotes Wildtyp-*Ctla4*-Gen und (ii) ein homozygotes Wildtyp-*Pdcd1-*Gen;
(III) Screenen des mindestens einen Kandidaten-Agens in einer Maus, umfassend (i) ein homozygotes Wildtyp-*Ctla4*-Gen und (ii) ein heterozygotes Loss-of-Function-Allel eines *Pdcd1-*Gens*;*
(IV) Screenen des mindestens einen Kandidaten-Agens in einer Maus, umfassend (i) ein heterozygotes Loss-of-Function-Allel eines *Ctla*4-Gens und (ii) ein homozygotes Wildtyp-*Pdcd1*-Gen; oder
(V) Screenen des mindestens einen Kandidaten-Agens in einer Maus, umfassend (i) ein heterozygotes Loss-of-Function-Allel eines *Ctla4*-Gens und (ii) heterozygotes Loss-of-Function-Allel eines *Pdcd1*-Gens.

13. Verfahren nach Anspruch 11 oder 12, wobei das mindestens eine Kandidaten-Agens gescreent wird auf
(I) seine Fähigkeit, die Entwicklung eines mit Immunität in Zusammenhang stehenden unerwünschten Ereignisses oder mit Immunität in Zusammenhang stehenden Zustands zu beschleunigen;
(II) seine Fähigkeit, den Schweregrad eines mit Immunität in Zusammenhang stehenden unerwünschten Ereignisses oder mit Immunität in Zusammenhang stehenden Zustands zu verschlimmern;
(III) seine Fähigkeit, die Penetranz eines mit Immunität in Zusammenhang stehenden unerwünschten Ereignisses oder mit Immunität in Zusammenhang stehenden Zustands in einer Population der Mäuse zu erhöhen; oder
(IV) seine Fähigkeit, ein mit Immunität in Zusammenhang stehendes unerwünschtes Ereignis oder einen mit Immunität in Zusammenhang stehenden Zustand abzuschwächen, wobei das Abschwächen eines mit Immunität in Zusammenhang stehenden unerwünschten Ereignisses oder eines mit Immunität in Zusammenhang stehenden Zustands des Weiteren als Vorbeugen der Entwicklung des mit Immunität in Zusammenhang stehenden unerwünschten Ereignisses oder eines mit Immunität in Zusammenhang stehenden Zustands definiert sein kann.

14. Verfahren nach einem der Ansprüche 11 bis 13, wobei das Kandidaten-Agens eine Anti-Krebs-Therapie; ein Pathogen, Stress, eine Verletzung und/oder eine Ernährungsweise; eine syngene Tumorzelle oder ein CTLA-4-Immunoglobulin-Fusionsprotein, ein Steroid, ein Agens, das eine spezifische Population von Immunzellen abbaut, ein Cytokin-modulierendes Agens oder ein immunsuppressives Agens ist.

15. Verfahren nach Anspruch 13 oder 14, wobei
(I) das mit Immunität in Zusammenhang stehende unerwünschte Ereignis eine Autoimmunität ist;
(II) der mit Immunität in Zusammenhang stehende Zustand eine Autoimmunerkrankung ist;
(III) das mit Immunität in Zusammenhang stehende unerwünschte Ereignis oder der mit Immunität in Zusammenhang stehende Zustand chronisch oder akut ist; oder
(IV) das mit Immunität in Zusammenhang stehende unerwünschte Ereignis oder der mit Immunität in Zusammenhang stehende Zustand
(i) eine Entzündung ist, die akut oder chronisch sein kann;
(ii) eine Autoimmunität ist, die eine durch eine Checkpoint-Blockade-Therapie bei Menschen ausgelöste Autoimmunität darstellt oder die ein mit Immunität in Zusammenhang stehendes unerwünschtes Ereignis bei Menschen darstellt;
(iii) lymphozytäre Myokarditis, Endarteritis, exokrine Pankreas-Destruktion, pulmonale Vaskulitis, Fettgewebe-Atrophie, hepatische Entzündung, Atrophie der weiblichen Fortpflanzungsorgane, Entzündung des Gastrointestinaltrakts, Synovitis oder lymphozytäre Infiltration der Niere, der Speicheldrüse, der Tränendrüse oder des Magens ist; oder
(iv) kardiale Autoimmunität oder Pankreas-Autoimmunität ist, bevorzugt wobei die kardiale Autoimmunität Myokarditis wie fulminante Myokarditis ist, oder bevorzugt wobei die Pankreas-Autoimmunität Insulin-abhängiger Diabetes mellitus ist oder exokrine Pankreas-Destruktion oder Pankreasinselzellen-Destruktion umfasst.

## Revendications

1. Souris dont le génome comprend : (i) un allèle de perte de fonction hétérozygote d'un gène *Ctla4* et (ii) un allèle de perte de fonction homozygote d'un gène *Pdcd1**.***

2. Souris selon la revendication 1, dans laquelle la souris présente un bagage génétique C57BL/6J.

3. Souris selon les revendications 1 ou 2, dans laquelle la souris est une souris femelle ou une souris mâle.

4. Souris selon l'une quelconque des revendications 1 à 3, dans laquelle l'allèle de perte de fonction hétérozygote d'un gène *Ctla4* est en outre défini comme une insertion hétérozygote d'une cassette présentant une résistance à la néomycine dans l'exon 3 du gène *Ctla4.*

5. Souris selon l'une quelconque des revendications 1 à 4, dans laquelle l'allèle de perte de fonction homozygote du gène *Pdcd1* est en outre défini comme une délétion homozygote des exons 2 et 3 du gène *Pdcd1.*

6. Souris selon l'une quelconque des revendications 1 à 5, dans laquelle la souris souffre d'auto-immunité.

7. Souris selon la revendication 6, dans laquelle l'auto-immunité est une auto-immunité cardiaque ou une auto-immunité pancréatique, de préférence dans laquelle l'auto-immunité cardiaque est une myocardite telle qu'une myocardite fulminante, et/ou de préférence dans laquelle l'auto-immunité pancréatique est un diabète sucré insulino-dépendant, ou comprend une destruction d'exocrine pancréatique ou une destruction d'îlots pancréatiques.

8. Souris selon la revendication 6, dans laquelle l'auto-immunité est une myocardite lymphocytaire, une endartérite, une destruction d'exocrine pancréatique, une vascularite pulmonaire, une atrophie de tissu adipeux, une inflammation hépatique, une atrophie des organes reproducteurs féminins, une inflammation du tractus gastro-intestinal, une synovite ou une infiltration lymphocytaire du rein, de la glande salivaire, de la glande lacrymale ou de l'estomac.

9. Cellule isolée d'une souris selon l'une quelconque des revendications 1 à 8.

10. Cellule selon la revendication 9, dans laquelle la cellule est une cellule immunitaire, de préférence un lymphocyte T.

11. Procédé de criblage d'au moins un agent candidat chez la souris selon l'une quelconque des revendications 1 à 8, comprenant l'administration d'un ou plusieurs agents candidats à la souris, dans lequel le au moins un agent candidat est de préférence criblé pour son efficacité.

12. Procédé selon la revendication 11, comprenant en outre les étapes consistant à
(I) cribler le au moins un agent candidat chez une souris comprenant (i) un gène *Ctla4* homozygote de type sauvage et (ii) un allèle de perte de fonction homozygote d'un *gène* Pdcd1 ;
(II) cribler le au moins un agent candidat chez une souris comprenant (i) un gène *Ctla4* homozygote de type sauvage et (ii) un gène *Pdcd1* homozygote de type sauvage ;
(III) cribler le au moins un agent candidat chez une souris comprenant (i) un gène *Ctla4* homozygote de type sauvage et (ii) un allèle de perte de fonction hétérozygote d'un gène *Pdcd1* ;
(IV) cribler le au moins un agent candidat chez une souris comprenant (i) un allèle de perte de fonction hétérozygote d'un gène *Ctla4* et (ii) un gène *Pdcd1* homozygote *de* type sauvage ; ou
(V) cribler le au moins un agent candidat chez une souris comprenant (i) un allèle de perte de fonction hétérozygote d'un gène *Ctla4* et (ii) une perte de fonction hétérozygote d'un gène ***Pdcd1.***

13. Procédé selon la revendication 11 ou 12, dans lequel le au moins un agent candidat est criblé pour
(I) sa capacité à accélérer le développement d'un événement indésirable lié à l'immunité ou d'une affection liée à l'immunité ;
(II) sa capacité à aggraver la gravité d'un événement indésirable lié à l'immunité ou d'une affection liée à l'immunité ;
(III) sa capacité à augmenter la pénétration d'un événement indésirable lié à l'immunité ou d'une affection liée à l'immunité dans une population de souris ; ou
(IV) sa capacité à atténuer un événement indésirable lié à l'immunité ou d'une affection liée à l'immunité, dans lequel l'atténuation d'un événement indésirable lié à l'immunité ou d'une affection liée à l'immunité peut être en outre définie comme la prévention du développement de l'événement indésirable lié à l'immunité ou de l'affection liée à l'immunité, ou comme la diminution de la gravité de l'événement indésirable lié à l'immunité ou de l'affection liée à l'immunité.

14. Procédé selon l'une quelconque des revendications 11 à 13, dans lequel l'agent candidat est une thérapie anticancéreuse ; un agent pathogène, un stress, une blessure, et/ou un régime alimentaire ; une cellule tumorale syngénique ; ou une protéine de fusion d'immunoglobuline *CTLA-4,* un stéroïde, un agent qui épuise une population spécifique de cellules immunitaires, un agent de modulation de cytokine, ou un agent immunosuppresseur.

15. Procédé selon la revendication 13 ou 14, dans lequel
(I) l'événement indésirable lié à l'immunité est une auto-immunité ;
(II) l'affection liée à l'immunité est une affection auto-immune ;
(III) l'événement indésirable lié à l'immunité ou l'affection liée à l'immunité est chronique ou aigu(ë) ; ou
(IV) l'événement indésirable lié à l'immunité ou l'affection liée à l'immunité est
(i) une inflammation qui peut être aiguë ou chronique,
(ii) une auto-immunité qui représente une auto-immunité induite par un traitement de blocage de point de contrôle chez l'homme ou représente un événement indésirable lié à l'immunité chez l'homme ;
(iii) une myocardite lymphocytaire, une endartérite, une destruction d'exocrine pancréatique, une vascularite pulmonaire, une atrophie de tissu adipeux, une inflammation hépatique, une atrophie des organes reproducteurs féminins, une inflammation du tractus gastro-intestinal, une synovite ou une infiltration lymphocytaire du rein, de la glande salivaire, de la glande lacrymale ou de l'estomac ; ou
(iv) une auto-immunité cardiaque ou une auto-immunité pancréatique, l'auto-immunité cardiaque étant de préférence une myocardite telle qu'une myocardite fulminante, ou l'auto-immunité pancréatique étant de préférence un diabète sucré insulino-dépendant, ou comprenant une destruction d'exocrine pancréatique ou une destruction d'îlots pancréatiques.
